# EUROPEAN PATENT SPECIFICATION

(11) **EP 0 431 072 B1**
(45) Date of publication and mention of the grant of the patent: **23.10.1996**
(21) Application number: 89910518.3
(22) Date of filing: 25.08.1989
(51) Int. Cl.: C12N 15/86

(54) **RECOMBINANT FOWLPOX VIRUS**
REKOMBINANTES GEFLÜGELPOCKENVIRUS
AVIPOXVIRUS DE RECOMBINAISON

(30) Priority: 26.08.1988 US 237285
(43) Date of publication of application: 12.06.1991
(73) Proprietor: APPLIED BIOTECHNOLOGY, INC., Cambridge Massachusetts 02142 (US)
(72) Inventor: COHEN, Lawrence, Kenneth, Brighton, MA 02135 (US); PANICALI, Dennis, L., Acton, MA 01720 (US)
(74) Representative: Holdcroft, James Gerald, Dr.
(86) International application number: PCT/US89/03701
(87) International publication number: WO 90/02191

(56) References cited:
- EP-A- 0 308 220
- EP-A- 0 314 569
- WO-A-86/00528
- WO-A-89/03429
- Technological Advances in Vaccine Development, 1988, L. Lasky (ED), Alan R. Liss, Inc. (NEW YORK,US) UCLA Symposia on Molecular and Cellular Biology New Series, vol. 84; J. TAYLOR et al.
- Israelian Journal of Veterinary Medicine, Vol. 42, No. 2, 1986; M.M. BINNS et al.: "Prospects for a Novel Genetically Enigineered Vaccine Against Infectious Bronchitis", pages 124-127

## Description

### Background

Fowlpox virus (FPV) is the archetypal member of the avian poxviruses and the causative agent of pox in poultry (Woodruff, A.M., and E.W. Goodpasture (1931) Am. J. Pathol. 7:209-222; Woodruff, C.E., and E.W. Goodpasture (1929) Am.. J. Pathol. 5:1-10; Woodruff, C.E., and E.W. Goodpasture (1930) Am. J. Pathol. 6:713-720). The virus particle is brick-shaped with dimensions of 260 x 350 nm and possesses the typical poxvirus structure. An outer membrane system encloses the lateral bodies and the biconcave core containing the viral genome which has been estimated at 200-240 x 10⁶ daltons (Gafford, L.G. and C.C. Randall (1967) J. Mol. Biol. 26:303-310).

Pox of birds is prevalent world-wide but is not considered a public health problem since the host-range of the avian poxviruses is limited to birds and excludes mammals (Tripathy, D.N. and G.H. Cunningham (1984) Avian Pox, Chapter 23, pp. 524-534, in Diseases of Poultry, 8th ed. M.S. Hofstad ed.). Chickens of all ages are susceptible to the disease and while mortality is usually low, infection causes a temporary decrease in egg production and a significant reduction in the growth rate of young birds. FPV infection most often occurs by mechanical transmission to injured or lacerated skin although the virus can also be transmitted by mosquitoes (DaMassa, A.J. (1966) Avian Dis. 10:57-66). After an incubation period of 4 to 10 days, the disease manifests itself as one or a combination of three forms: (1) cutaneous lesions of featherless areas; (2) diphtheric lesions of the mouth; and (3) coryzal lesions of nasal passages (Tripathy, D.N., and C.H. Cunningham (1984) Avian Pox, Chapter 23, pp. 524-534, in Diseases of Poultry, 8th ed. M.S. Hofstad ed.). In uncomplicated infections the disease lasts 3-4 weeks and results in life-long immunity in the bird, a result of both humoral and cell-mediated responses (Tripathy, D.N., and L.E. Hanson (1975) Am. J. Vet. Res. 36:541-544).

Attenuated strains of FPV are currently being used by the poultry industry as vaccines to control the incidence of pox in chickens and turkeys. The live viral vaccine, which results in life-long immunity, is prepared on the chorioallantoic membrane of the chicken embryo or from chicken embryo fibroblast cell cultures. Vaccinations are administered to chicks as young as one day old either orally or by pricking the web-wing (Tripathy, D.N., and C.H. Cunningham (1984) Avian Pox, Chapter 23, pp. 524-534, in Diseases of Poultry, 8th ed. M.S. Hofstad ed.; Mayr, A., and K. Danner (1976) Develop. biol. Standasr. 33:249-259). The FPV vaccine has been used in combination with a vaccine for Marek's Disease Virus to protect against both diseases with a single inoculation (Siccardi, F.J. (1975) Avian Dis. 19:362-365).

Laboratory analyses of FPV have concentrated on the characterization of the growth of the virus in birds, the chorioallantoic membrane (CAM) of developing embryos, and tissue culture cells. Replication in the dermal or follicular epithelium of birds is similar to that on the CAM (Tripathy, D.N., and C.H. Cunningham (1984) Avian Pox, Chapter 23, pp. 524-534, in Diseases of Poultry, 8th ed. M.S. Hofstad ed.). After adsorption, penetration and uncoating of the virus, a host response consisting of hyperplasia and the replication of cellular DNA occurs for the first 72 hours and generally results in a 2.5 fold increase in the number of cells (Cheevers, W.P., and C.C. Randall (1968) Proc. Soc. Exp. Biol. Med. 127:401-405; Cheevers, W.P., D.J. O'Callaghan, and C.C. Randall (1968) J. Virol. 2:421-429). Viral DNA replication which is preceded by early protein synthesis occurs primarily between 60 and 96 hours post-infection and is followed by the synthesis of late proteins. The assembly of infectious virions occurs between 72 and 96 hours (Cheevers, W.P., and C.C. Randall (1968) Proc. Soc. Exp. Biol. Med. 127:401-405; Cheevers, W.P., D.J. O'Callaghan, and C.C. Randall (1968) J. Virol. 2:421-429).

The growth of FPV on tissue culture cells has been achieved on chicken embryo fibroblast cells, duck embryo fibroblast cells, and chicken embryo dermal cells (Gafford, L.G., and C.C. Randall (1976) Virology 33:112-120; Baxendale, W. (1971) Vet. Rec. 88:5.10; El-Zein, A., S. Nehme, V. Ghoraib, S. Hasbani, and B. Toth (1974) Avian Dis. 18:495-506). In each case, the viral cycle is similar and appears to be quicker than that defined in birds. In the CED cells DNA replication commences between 12 and 16 hours, and infectious virus particles first appear at 16 hours and continue to increase in number until 48 hours post-infection (Prideaux, C.T., and D.B. Boyle (1987) Arch. Virol. 96:185-199).

Investigations of the organization of the FPV genome have recently been reported by a number of laboratories. The thymidine kinase gene was identified by complementation of a thymidine kinase negative vaccinia virus, and the DNA sequence of this gene has been determined (Boyle, D.B., and B.H. Coupar (1986) J. Gen. Virol. 67:1591-1600; Boyle, D.B., B.H. Coupar, A.J. Gibbs, L.J. Seigman, and G.W. Both (1987) Virology 156:355-365). Importantly, this study demonstrated the functional cross-reactivity of FPV and vaccinia virus promoters. The FPV DNA polymerase gene was identified by amino acid homology to the vaccinia virus polymerase, and the DNA sequence of this gene was also reported (Binns, M.M., L. Stenzler, F.M. Tomley, J. Cambell, and M.E.G. Boursnell (1987) Nucleic Acid Research 16:6563-6573). Twenty-one polypeptides associated with the FPV infectious cycle have been detected by metabolic labeling of infected chicken dermal cells, and a 3.1 kb fragment of the FPV genome which demonstrates nucleic acid homology with the vaccinia virus HindIII J fragment has been identified (Prideaux, C.T., and D.B. Boyle (1987) Arch. Virol. 96:185-199; Drillien, R., Spehner, D., Villeval, D., and J.-P. LeCocq (1987) Virology 160:203-209).

Vaccinia virus, the archetypal member of the orthopox viruses, was employed as a vaccine for the successful worldwide erradication of smallpox. The success of the program is attributable in part to: (1) the high levels of both cellular and humoral immunity achieved in response to infection with vaccinia virus; (2) the stability of the virus; (3) the ease of administration of the vaccine; and (4) the relative safety of the innoculation.

Paoletti et al. have developed a technique known as in vivo recombination (IVR) which allows the insertion by site-specific recombination of foreign DNA into the vaccinia virus genome (U.S. Patent No. 4,603,112), and has led to the use of vaccinia virus as a eukaryotic expression vector for creating live recombinant vaccines. A number of recombinant vaccinia virus have been created expressing either single or multiple genes encoding specific foreign viral antigens and upon vaccination have been shown to protect against challenge with the correlate pathogens.

Boyle, D. et al. have disclosed recombinant FPV containing foreign DNA within a region of the viral genome which is nonessential for growth in tissue culture. International Patent Application PCT/AU87/ 00323, Boyle and Coupar (1988) Virus Res. 10:343. Vaccinia virus promoters are used to express the DNA in FPV.

Recently, several groups have published the construction of FPV recombinants. Noboru et al., (EPO 284,416, filed March 25, 1988) disclose a number of genomic insertion sites which are nonessential for FPV growth in tissue culture, using the E. coli lacZ gene under the control of a vaccinia promoter. Paoletti (PCT/US88/02816, filed August 24, 1988; Taylor et al., (1988) Vaccine 6: 497-503, 504-508) describes vectors for producing FPV recombinants using various vaccinia promoters for the expression of genes encoding foreign antigens, including the rabies G protein, turkey influenza hemagglutinin and avian bronchitis virus spike protein. The vectors are designed to insert the foreign gene at genomic insertion sites which are nonessential for growth in tissue culture. In several cases, partial protection of the appropriate target animal, avian or mammalian, against challenge with the pathogenic virus was demonstrated. In addition, a FPV promoter from a gene encoding an abundant FPV protein was identified. Binns et al. (PCT/GB88/ 00922) disclose the identification of a number of FPV promoters using a transient assay with β-galactosidase. Drillien and Spehner (EPO 314,569, filed October 26, 1988) disclose the construction of FPV recombinants containing a gene encoding the measles F protein under the control of a vaccinia promoter. The gene was inserted into the FPV genome at a site nonessential for growth in tissue culture.

Only Paoletti describes inoculation of animals with recombinant FPV. No report discloses the effect of insertions at genomic sites, nonessential in tissue culture, on the pathogenicity, immunogenicity or transmissibility of recombinant FPV in a host animal as compared to the parental FPV. Insertions into certain genomic sites, although nonessential in tissue culture, may have an undesirable effect on viral virulence or immunogenicity in the immunized animal. In vaccinia virus, for example, a commonly used genomic insertion site is the thymidine kinase (TK) gene. However, inactivation of this gene results in a recombinant vaccinia virus which is ten-fold less immunogenic than the parental virus which contains the TK gene (L. Payne, personal communication). Insertions at other sites in the vaccinia genome do not affect immunogenicity. Thus, to make an effective recombinant vaccine, the choice of insertion site can be critical.

### Summary of the Invention

This invention pertains to recombinant FPV which contain and express foreign DNA under the direction of a FPV or other pox viral promoter, to methods of producing the recombinant FPV and to the use of recombinant FPV for the manufacture of live viral vaccines.

Recombinant FPV capable of expressing foreign antigens are produced by integrating into the fowlpox viral genome a gene or genes encoding a foreign antigen(s). This foreign DNA sequence is inserted in a region of the FPV genome homologous to the BamHI J fragment of the fowlpox virus and which is nonessential for replication of the pox virus. The insertion of foreign DNA into this region does not substantially reduce the immunogenicity and does not increase the pathogenicity or transmissibility of the recombinant fowipox virus as compared to the parental FPV in a host animal. A substantial reduction in immunogenicity is one which would impair the ability of the host animal to elicit a protective immune response to the recombinant fowlpox virus. The foreign DNA is inserted into the genome in association with a FPV or other pox viral promoter to direct the expression of the foreign DNA.

The foreign DNA sequence is integrated into the FPV genome by an in vivo recombination event between an intermediate DNA vector carrying the foreign DNA sequence and the FPV genome. The intermediate DNA vector contains the foreign DNA sequence linked to a fowlpox or other viral promoter located between DNA sequences homologous to the BamHI J fragment of the FPV genome which is nonessential for replication of FPV. Thus, the vector comprises:
a. a prokaryotic origin of replication;
b. one or more FPV or other pox viral promoters;
c. one or more DNA sequences encoding antigens, each DNA sequence being under the direction of a separate FPV or other pox viral promoter; and
d. DNA sequences of the FPV into which the gene encoding the antigen is to be integrated, the DNA sequences flanking the promoter and structural gene at both the 5' and 3' ends, the DNA sequences being homologous to the region of the FPV genome where the promoter(s) and foreign DNA sequence(s) are to be inserted. In addition, insertion into this genomic region should be nonessential for growth of the recombinant fowlpox virus in tissue culture and should not result in a recombinant FPV which is substantially less immunogenic or more pathogenic to the host animal than is the parental fowlpox virus. The flanking DNA sequences are homologous to DNA sequences of the BamHI J fragment of FPV.

In preferred form, the DNA vector for recombination with FPV also contains a gene which encodes a selectable marker which permits selection of viral recombinants containing the inserted foreign DNA. Thus, the vector will contain these additional elements located between the flanking FPV sequences:
e. a second pox viral promoter (preferably, a FPV promoter); and
f. a gene encoding a selectable marker, the gene being under the direction of the second pox viral promoter.

Recombination of the DNA vector with FPV is achieved in an appropriate eukaryotic host cell. Appropriate host cells for in vivo recombination are eukaryotic cells which are 1) transfectable by the DNA vector and 2) infectable by FPV. The host cell is infected with the FPV and then transfected with the DNA vector. Virus is allowed to replicate in the host cell and recombination occurs in vivo resulting in insertion of the foreign DNA into the FPV genome. The recombinant viral progeny is isolated away from the wild type virus. When a selectable marker has been co-integrated with the foreign DNA sequence, expression of the selectable marker provides a basis for selection of recombinant virus containing integrated foreign DNA. Other methods of selection include detection of integrated DNA by hybridization with homologous DNA probes or selection for absence of the product of the viral gene into which the DNA vector has been inserted.

The recombinant virus is a virus which expresses in tissue culture and in an inoculated host the foreign antigen of interest. The virally-expressed antigen will trigger cell-mediated and humoral immunity against the virus from which the antigen is derived.

There are a number of advantages to creating a recombinant FPV expressing foreign genes for use as live vaccines in fowl and perhaps other animals. Vaccination with a live virus would stimulate cell-mediated and humoral immunity. The proteins that are expressed are expected to be appropriately modified, and if the required signals are present, they may also be localized to the proper regions of the cell or cellular membrane (Stephens et al., EMBO J, 5:237 (1986)). In contrast to subunit vaccines, live virus vaccines stimulate both cell-mediated and humoral immunity. Additionally, a single FPV isolate could serve as a polyvalent vaccine against one or more pathogens as has been demonstrated in vaccinia virus (Perkus et al., Science, 229:981 (1985)). FPV vaccines can be manufactured inexpensively. The vaccines can also be administered relatively simply. Finally, a recombinant vaccine utilizing FPV would avoid the problems associated with vaccinating with live attenuated or killed pathogens: these pathogens may not be properly killed or can revert virulent forms.

### Brief Description of the Figures

Figure 1 A-C show two plasmids designated, pAbT 405 and pAbT 4523, containing vaccinia virus promoters and one plasmid designated, pAbT 2102, containing a FPV promoter directly upstream of the lacZ from E. coli.

Figures 2 A-C show plasmids containing the promoter-lacZ cassette derived from pAbT 4523 flanked by FPV DNA sequences to direct insertion into the genome by homologous recombination.

Figures 3A-3D show hybridization analysis and predicted genomic structures for two recombinant FPV with insertions at two different sites.

Figures 4 A-D show the plasmids into which fragments of FPV genome DNA were cloned to identify FPV promoter elements.

Figures 5 A-C show the DNA sequence of FPV promoters C₁ and C₂ and the DNA sequence of two modified C₁ promoters.

Figures 6 A-C show generalized plasmids for in vivo recombination with FPV.

Figures 7A-C show generalized plasmids for in vivo recombination with FPV.

Figure 8 shows the plasmid into which the gene encoding the spike protein of avian infectious bronchitis virus strain m41 was inserted.

Figure 9 shows the plasmid into which the Eimeria GX3262 gene was inserted.

### Detailed Disclosure of the Invention

### 1. Genes for integration into pox virus

Foreign genes for integration into the genome of a FPV in expressible form can be obtained by any conventional technique for isolating a desired gene. The genes can be derived from any organism, including bacteria, viruses or other microorganisms, for which a FPV-based live vaccine is desired. These genes will be derived from pathogens which are important to the poultry industry and includes those pathogens for which vaccines of variable efficacy already exist, namely: infectious bronchitis virus, infectious bursal disease virus, reovirus, Marek's disease virus, Newcastle disease virus, laryngotracheitis virus, and avian encephalomyelitis virus. The genes will also be derived from poultry pathogens for which no vaccines currently exist despite the need to control their spread. This list includes: Eimeria species which cause coccidiosis (e.g., Eimeria tenella), salmonella gallinarum, Salmonella pullorum, Salmonella typhimurium, Staphylococcus aureus, Aspergillus flavus, Escherichia coli, Mycoplasma gallisepticum, Mycoplasma gallinarum, Mycoplasma synoviae, RNA lymphoid leukosis virus, avian influenza, and hemorrhagic enteritis virus.

For purposes of a vaccine, genes of interest are those which encode immunogenic proteins of a pathogenic organism. In many cases, these are protein components of surface structures such as the bacterial cell wall or viral envelope. In appropriate instances, immunogenic fragments or subunits of the proteins may be used.

For organisms which contain a DNA genome, the genes encoding an antigen of interest are isolated from the genomic DNA; for organisms with RNA genomes, the desired gene may be isolated from cDNA copies of the genome. If restriction maps of the genome are available, strategies can be designed for cleaving genomic DNA by restriction endonuclease digestion to yield DNA fragments that contain the gene of interest. In some cases, desired genes may have been previously cloned and thus, the genes can be obtained from the available clones. Alternatively, if the DNA sequence of the gene is known, the gene can be synthesized by any of the conventional techniques for synthesis of deoxyribonucleic acids (e.g., the phosphate or phosphite triester techniques).

Genes encoding an antigen of interest can be amplified by cloning the gene into a bacterial host. For this purpose, various prokaryotic cloning vectors can be used. Examples are plasmids pBR322 and pEMBL.

The genes encoding the antigen of interest can be prepared for insertion into the DNA vectors designed for recombination with FPV by standard techniques. In general, the cloned genes can be excised from the prokaryotic cloning vector by restriction enzyme digestion. In most cases, the excised fragment will contain the entire coding region of the gene. The DNA fragment carrying the cloned gene can be modified as needed, for example, to make the ends of the fragment compatible with the insertion sites of the DNA vectors used for recombination with FPV, then purified prior to insertion into these vectors at restriction endonuclease cleavage sites (cloning sites) as described below.

### 2. DNA vectors for recombination with FPV

According to the method of this invention foreign genes which encode immunogenic proteins are inserted into the genome of FPV so as to allow them to be expressed by the FPV along with the expression of the normal complement of FPV proteins (except for the FPV protein encoded by the gene into which the foreign DNA is inserted). This is accomplished by first constructing a DNA vector for recombination with FPV which contains the foreign gene or genes of interest flanked by FPV sequences. The flanking FPV sequences can be derived from any FPV DNA region nonessential for replication; these allow the vector to recombine with the virus in vivo at a specific region in the viral genome. This recombination results in integration of the foreign DNA into the genome to produce a recombinant FPV containing the foreign gene or genes.

Preferred DNA vectors for integration of a foreign gene in expressible form into the FPV genome contain the following elements:
a. a pox viral promoter linked to:
b. a DNA sequence containing a multiple cloning site for insertion of foreign DNA;
c. DNA sequences flanking the construct of elements a and b, the flanking sequences being homologous to a region of the FPV genome into which elements a and b are to be inserted;
d. a replicon for vector replication in a prokaryotic host; and
e. a gene encoding a selectable marker or indicator for selection of the vector in transformed prokaryotic hosts.

The multiple cloning site comprises recognition sites for several restriction enzymes which allow different modes of insertion of foreign DNA. An example sequence containing a multiple cloning site is: GGATCCCCGGGTACCGAGCTCGAATTC, which contains the recognition sequences and cleavage sites for the restriction endonuclease enzymes BamHI, SmaI, KpnI, SacI and EcoRI. Sequences containing additional or different recognition sites can be used. The cloning site is located adjacent to and downstream of a pox viral promoter such that an inserted gene can be placed under transcriptional control of the promoter.

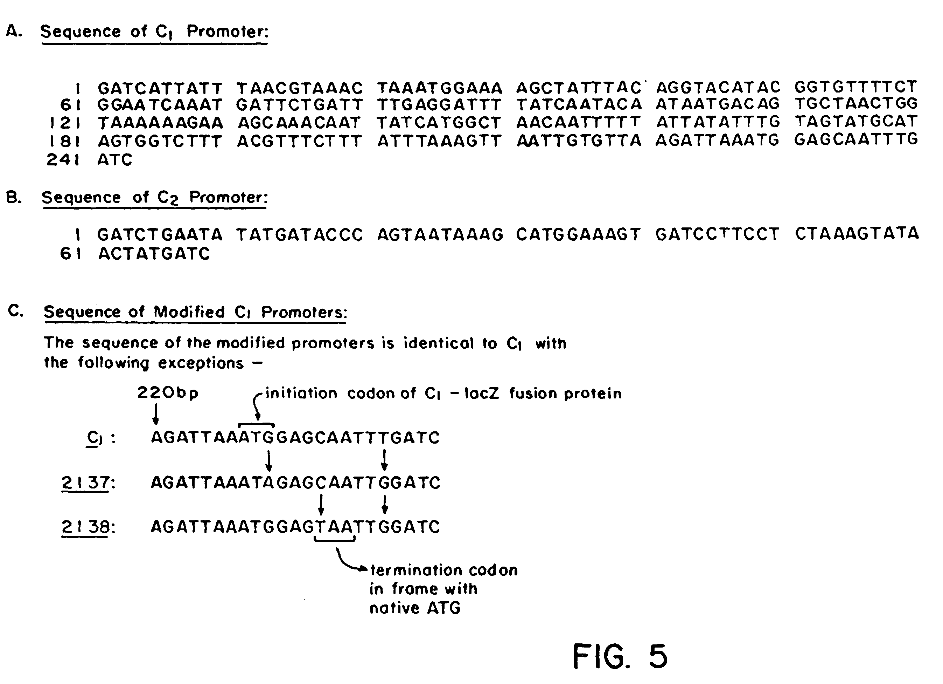

The pox viral promoter controls expression of the foreign gene inserted at the cloning site. The preferred promoter is derived from FPV but other pox viral promoters (e.g., vaccinia promoters) can also be used. FPV promoters are DNA sequences which direct messenger RNA synthesis from FPV genes during a virus infection. Such promoters can be isolated from the FPV genome or can be constructed by DNA synthesis techniques. Promoters vary in strength of activity and in time of expression during the FPV replication cycle; these parameters can be altered by mutation of the promoter sequence. Especially preferred are the FPV C₁ and C₂ promoters having the sequences shown in Figures 5A and 5B. Modified versions of the C₁ promoter such as those shown in Figure 5C can also be used.

The sequences flanking the construct of elements a and b (the pox viral promoter and adjacent cloning site) are homologous to the BamHI J fragment of the FPV genome or a portion thereof sufficient for recombination. Thus, recombination and integration of foreign DNA will occur at this site and the insertion will not abolish viral replication. Preferably, insertion into this region will not substantially reduce the immunogenicity or increase the pathogenicity or transmissibility of the recombinant fowlpox virus as compared to the parental fowlpox virus in poultry or other animals.

The FPV region for recombination and insertion of foreign DNA is the BamHI J fragment of FPV. Recombination can be directed to the BgIII site within this fragment. This can be accomplished by employing, as 5' flanking sequences in the vector, DNA homologous to the region of the BamHI J fragment which is 5' of the BgIII site and, as 3' flanking sequences, DNA homologous to the region of the BamHI J fragment which is 3' of the Bg1II site.

The replicon for replication in a prokaryotic host and the gene encoding the selectable indicator or marker allow the vector to be selected and amplified in a prokaryotic host such as E. coli to provide ample quantities of the vector DNA for eventual transfection of eukaryotic host cells for recombination. The replicon can be obtained from any conventional prokaryotic vector such as pBR322 or pEMBL. The selectable marker can be a gene conferring antibiotic resistance (e.g. ampicillin, chloramphenicol, kanamycin or tetracycline resistance).

Preferred vectors contain genetic elements which permit positive selection or identification of recombinant FPV i.e., those viruses which have recombined with the vector and, as a result, have acquired the foreign DNA sequences. These elements comprise a gene encoding a selectable marker or indicator and a pox viral promoter which controls expression of the gene in the recombinant virus. The promoter and marker or indicator gene are located between the flanking FPV sequences so that the elements which allow for selection or identification and the foreign gene of interest are co-integrated into the FPV genome. Recombinant FPV can then be selected based upon expression of the marker or indicator.

A preferred gene for identification is the E. coli lacZ gene which encodes the enzyme B-galactosidase. Methods of identification based upon expression of this enzyme are discussed below. Selection methods include any drug resistance selection, for example, the selection that is provided by the gene encoding xanthine-guanine phosphoribosyl transferase, which confers resistance to mycophenolic acid or that encoding neomycin phosphotransferase, an enzyme which confers resistance to G418 (Franke et al., (1985) Mol. Cell. Biol. 5: 1918; Falkner, F.G. and B. Moss (1988) J. Virol. 62:1869.1856.)

Accordingly, a vector for recombination with FPV can contain:
a. an FPV or other pox viral promoter (e.g. the C₁ or C₂ FPV promoters of Figure 5 or modified versions of these promoters);
b. a multiple cloning site adjacent to the promoter;
c. a second pox viral promoter (preferably derived from FPV);
d. a gene encoding an identifiable marker (e.g. the E. coli lacZ gene) under the control of element c;
e. DNA sequences homologous to a region of the FPV genome into which elements a-d are to be inserted, the DNA sequences flanking the construct of elements a-d (e.g., DNA sequences homologous to the BamHI J fragment of FPV);
f. a replicon for replication in a bacterial host; and
g. a gene encoding a selectable marker under control of a prokaryotic promoter for selection of the vector in a prokaryotic host.

Two or more foreign DNA sequences can be inserted into FPV by a single in vivo recombination event. For this purpose, vectors are provided for introduction of two or more foreign DNA sequences into FPV. These vectors can be employed to produce recombinant FPV which express two or more different antigenic proteins to provide multivalent vaccines. The proteins can be derived from one or more organisms. Vectors for introduction of multiple foreign DNA sequences into FPV contain the elements as described above for a monovalent vector and one or more additional pox viral promoters (preferably FPV promoters), each followed by a multiple cloning site for insertion of one or more additional genes encoding immunogenic proteins of one or more organisms. The additional promoter(s) and multiple cloning site(s) are located between the flanking FPV sequences which direct recombination into the FPV genome. A vector for introduction of two or more foreign DNA sequences into a FPV can comprise any combination of two, three or more of the various FPV or other pox viral promoters, each followed by a multiple cloning site for the insertion of a different gene of interest. For example, a divalent vector can contain:
a. a first FPV or other pox viral promoter followed by a multiple cloning site appropriately situated so that any inserted DNA sequence is controlled by the promoter;
b. a second FPV or other pox viral promoter and adjacent cloning site; and
c. a third FPV or other pox viral promoter linked to a foreign DNA sequence encoding a selectable marker or indicator.

This construct is flanked by sequences of FPV which direct recombination. The first, second, and third promoters must be different promoters. There are several advantages to using different promoters in one vector. Different promoters may have varying strengths of activity and different times of expression during the viral life cycle. Separate promoters are also required to ensure the stability of the ensuing recombinant FPV. It has recently been demonstrated that tandem repeats of identical nucleic acid sequences are unstable in pox virus genomes, and are removed through homologous recombination during replication of the viral genomes (Ball, L.A. (1987) J. Virol. 61:1788-1795; and Spyropoulos, D.D., B.E. Roberts, D.L. Panicali, and L.K. Cohen (1988) J. Virol. 62:1046-1054). The parameters for optimal expression of different antigens may vary with each antigen and therefore different promoters may be optimal.

Because of the convenient multiple cloning sites, any isolatable gene(s) can be easily inserted into these monovalent and divalent in vivo recombination vectors.

### 4. In vivo recombination

The intermediate DNA vectors containing the DNA sequence of interest (and the marker or indicator gene) flanked by appropriate FPV sequences will undergo recombination with FPV which results in integration of the flanked genes into the FPV viral genome. This recombination will occur in a eukaryotic host cell. Appropriate host cells for recombination are cells which are 1) infectable by FPV and 2) transfectable by the DNA vector. Examples of such cells are chick embryo fibroblast (CEF), and chick embryo dermal (CED) cells.

The cells are first infected with FPV and then transfected with the intermediate DNA vector. Viral infection is accomplished by standard techniques for infection of eukaryotic cells with FPV. (See e.g., Paoletti et al., supra.) The cells can be transfected with the intermediate DNA vector by any of the conventional techniques of transfection. These include the techniques of calcium phosphate precipitation, DEAE dextran, electroporation and protoplast fusion. The preferred technique is the calcium phosphate precipitation technique.

After infection and subsequent transfection, the cells are incubated under standard conditions and virus is allowed to replicate during which time in vivo recombination occurs between the homologous FPV sequences in the intermediate vector and FPV sequences in the genome.

Recombinant viral progeny are then selected by any of several techniques. The presence of integrated foreign DNA can be detected by hybridization with a labeled DNA probe specific for the inserted DNA. Preferred techniques for selection, however, are based upon co-integration of a gene encoding a marker or indicator gene along with the gene of interest, as described above. A preferred indicator gene is the E. coli lacZ gene which encodes the enzyme beta-galactosidase. Selection of recombinant FPV expressing beta-galactosidase can be done by employing a chromogenic substrate for the enzyme. For example, recombinant viruses are detected as blue plaques in the presence of the substrate 5-bromo-4-chloro-3-indolyl-B-D-galactoside or other halogenated-indolyl-β-D-galactosides (e.g. BluoGal^{TM}).

### 5. Vaccines

Live recombinant FPV expressing immunogenic proteins from one or more pathogens can be used to vaccinate poultry susceptible to these pathogens. Recombinant FPV may also be useful to vaccinate animals other than poultry. These vaccines may be administered intradermally or by other routes appropriate to the recombinant virus used and the disease for which protection is desired. These may include among others, intra-muscular, subcutaneous and oral routes. Vaccination with live recombinant FPV is followed by replication of the virus within the host. During replication, the foreign DNA sequence(s) is expressed along with the normal complement of FPV genes. If the foreign DNA sequence encodes an antigen, the host will mount an immunological response, both humoral and cell-mediated, to the foreign antigen as well as to FPV itself. If the foreign antigen can stimulate a protective immunological response, then the host animal will be immune to infection by the corresponding pathogenic agent.

Live recombinant FPV containing and expressing one or more of the genes encoding immunogenic proteins of one or more pathogenic agents can serve as monovalent and divalent vaccines.

An additional advantage of utilizing recombinant FPV as live vaccines is that they express only selected antigens, preferably only those antigens sufficient to elicit a protective immune response to the corresponding pathogen. It is therefore possible to differentiate between host animals which have been vaccinated with the recombinant FPV and those which have been infected with the authentic, virulent, disease-causing agent.

The vaccinated host will develop antibodies only to the FPV and to the selected foreign antigen(s). By contrast, the actively infected host will generate a full complement of antibodies directed toward the pathogenic agent, including antibodies directed to specific antigens not present in the recombinant FPV. The presence of these additional antibodies, which can be detected using appropriate immunological tests (e.g., ELISA), is therefore diagnostic of a naturally occuring infection of the host with the pathogen.

The invention is illustrated by the following Exemplification.

### Exemplification

### MATERIALS AND METHODS

### 1. Cells.

Primary chicken embryo fibroblast (CEF) and chicken embryo dermal (CED) cells were prepared by published procedures (Rein, A., and H. Rubin (1968) Exp. Cell Res. 49:666; Silim, A., M.A.S.Y. El Azhary, and R.S. Roy (1982) Avian Dis. 26:182.185). The fibroblast cultures were maintained in Dulbecco's Modified Eagle Media (DME) supplemented with 10% calf serum (CS), and the dermal cells were maintained in Minimum Essential Media (MEM) containing 5% fetal calf serum (FCS). The fibroblast cultures were maintained for a maximum of 3 passages in tissue culture and the dermal cells for a maximum of 6 passages. All cells were grown at 37°C and under 5% CO₂.

### 2. Virus Strains.

A FPV vaccine strain obtained from Schering-Plough and designated for research purposes only, was employed exclusively in these studies. The strain was plaque purified twice on both CEF and CED monolayers before use.

### 3. Amplification and Purification of FPV.

Viral stocks were prepared on CED monolayers by infection at a multiplicity of infection (moi) of 0.01 and replication was allowed to proceed for 5 days at 37°C and 5% CO₂. Infected cells were scraped directly into the culture media and virus was released by multiple cycles of freezing and thawing.

The amplification of single plaque isolates was conducted by infection of 2 x 10⁶ cells in a confluent monolayer with one-half of the plaque pick. After five days of replication, the progeny virus were harvested, released by multiple cycles of freezing and thawing, and one-third of that material was used to inoculate 1 x 10 cells. Amplification was again allowed to proceed for five days, and the resulting virus was harvested by scraping the cells into the culture media, and released by multiple cycles of freezing and thawing. A viral stock prepared in this fashion results in a stock containing approximately 1 X 10⁸ pfu.

Partially purified virus was prepared, when necessary, by centrifugation by a procedure developed for the purification of vaccinia virus (Joklik, W.K. (1962) Virology 13:9-18).

### 4. Plaque Assay of FPV.

Plaque formation of FPV was accomplished on monolayers of CED cells which were seeded at a density of 2 x 10⁶ cells per 60 mm tissue culture dish. Viral suspensions were allowed to adsorb to the cells in 1.0 ml of MEM containing 2% FCS for 90 minutes, and were removed by aspiration and replaced with 5.0 mls of 0.6% agarose containing DME and 2% CS. The plaques formed at 37°C and 5% CO₂ were generally visible after 48 hours, and were stained for 12 to 48 hours with an additional agarose overlay containing 0.001% neutral red. The Beta-galactosidase activity following plaque formation was detected with an agarose overlay containing DME and 300 µg/ml of 5-bromo-4-chloro-3-indolyl-β-D-galactopyranoside (X-gal).

### 5. FPV-Based Transient Expression Assays.

The FPV-based transient assay was based directly on the assays which have been developed for use with vaccinia virus (Panicali, D.L., A. Grzelecki, and C. Huang (1986) Gene 193-199; Cochran, M.A., M. Mackett, and B. Moss (1985) Proc. Natl. Acad. Sci. USA 82:19-23). Confluent monolayers of 2 x 10⁵ CEF cells were infected with FPV at an moi of 1 for 60-90 minutes. After this, the inoculum was removed and replaced with growth medium. About 2.5 µg of a plasmid DNA containing a putative FPV promoter element directly upstream of the E. coli lacZ was precipitated from 62.5 µl of N-2-hydroxyethylpiperazine-N'-2-ethanesulfonic acid (HEPES)-buffered saline (20 mM HEPES, 150 mM NaCl, 0.7 mM Na₂HPO₄, 5 mM KC1, and 6mM glucose [pH 7.0]) by the addition of 3.9 µl of 2 M CaCl₂ at room temperature for about 30 to 45 minutes. This precipitated DNA was added directly to the growth media 45 minutes after the removal of the viral inoculum. Viral replication and transient expression were allowed to proceed for approximately 24 hours, after which time the media and transfected DNA were removed and replaced with 1 mg/ml o-nitrophenyl-β-D-galactosidase (ONPG) in phosphate buffered saline (PBS, 2.7 mM KC1, 1.5 mM KH₂PO₄, 137 mM NaCl, and 8.1 mM Na₂PO₄). Incubation in the presence of the chromogenic indicator was conducted at 37°C for times varying between 1 to 12 hours after which the production of nitrophenol was measured by absorbance at 420 nm.

### 6. Conditions for the Formation of Recombinant FPV

Confluent monolayers of 2 x 10⁶ CEF cells in 60 mm tissue culture dishes were infected at an moi of 0.1 for about 60 to 90 minutes in MEM containing 2% FCS. Following adsorption of the virus, the inoculum was removed and replaced with 5.0 ml of growth medium. Twenty µg of plasmid DNA in 500 µl of HEPES-buffered saline was precipitated from the buffer by the addition of 31 µl of 2 M CaCl₂ at room temperature for 30 to 45 minutes. The precipitated plasmid DNA was added directly to the growth medium. Viral replication and recombination were allowed to proceed for an additional 72 hours. The viral progeny were harvested by scraping the CEF cells directly into the growth media, released by multiple cycles of freezing and thawing, and titered on confluent monolayers of CED cells.

### 7. Identification of Recombinant FPV Using A Single Cell Beta-Galactosidase Assay.

Monolayers consisting of 1 x 10⁶ CED cells in 35 mm dishes were infected with recombinant FPV at an moi of 1.0 in MEM containing 2% FCS. After 60 to 90 minutes the FPV inoculum was removed and replaced with 3.0 ml of growth medium. Infection was allowed to proceed for an additional 24 hours. Growth media was removed, the monolayers washed 3 times in 3.0 ml of PBS, and the cells fixed in 0.5% glutaraldehyde in PBS for 5 to 15 minutes at room temperature. Following fixation, the glutaraldehyde was removed by three washes with PBS at room temperature for 3 to 5 minutes each. The cells were stained for beta-galactosidase activity at 37°C for 2 to 6 hours with a solution of 1 mg/ml X-gal in 5 mM potassium ferricyanide, 5 mM potassium ferrocyanide, and 1 mM MgSO₄ in PBS. Following staining, the cells were washed extensively with PBS and examined microscopically for the presence of stained cells. Cells which are stained blue are indicative of beta-galactosidase activity.

### 8. Preparation of Viral Genomic DNA.

FPV genomic DNA was prepared by infecting a confluent monolayer of CED cells with FPV at an MOI of 10, incubating overnight at 39°C until 100% CPE was observed. The infected cells were scraped directly into the growth medium and were harvested by centrifugation at 3000 rpm for 10 minutes in a clinical centrifuge. The supernatant was discarded and the cell pellet washed 3 times by resuspension in 10 ml of PBS and recentrifuged. The final washed cell pellet was resuspended in 1.8 ml of 10 mM Tris-Cl (pH7.8), 5 mM EDTA, and 10mM KC1, and incubated on ice for about 10 minutes with intermittent vortexing. One µl of 2-mercaptoethanol and 200 µl of a solution of 10% Triton X-100 were added and the cells were lysed for an additional 10 minutes on ice with intermittent vortexing. Nuclei and large debris were removed by contrifugation at 2000 rpm for 10 minutes in a clinical centrifuge. This centrifugation was repeated one time. The supernatant containing the partially purified virus was treated at 37° C for two hours with 10 µl of a 20 mg/ml solution of proteinase K in H₂O, 40 µl of 5 M NaCl, and 100 µl of 10% SDS. The supernatant was then extracted twice with phenol/chloroform (1/1:v/v). The genomic DNA was then precipitated by the addition of one tenth the volume of 3M sodium acetate and 2 volumes of ethanol at -20°C for about 30 minutes. The nucleic acid was collected by centrifugation at 12000 rpm for 10 minutes in a Sorvall SS-34 rotor, and, after drying, was resuspended in 50 µl of 10 m, Tris-Cl (pH 8.0), 1 mM EDTA.

### 9. Hybridization Analysis.

Viral genomic DNA was digested with restriction endonuclease BamHI for 4 hours and the resulting fragments were resolved on 1% agarose gels containing 40 mM Tris-acetate (pH8.0), 2 mM EDTA. The fragments were transferred to nitrocellulose and analyzed by hybridization to the appropriate radiolabelled DNA by standard procedure (Maniatis, T., E.F. Fritsch, and J. Sambrook (1982) Molecular Cloning, A Laboratory Manual).

### 10. Construction of Plasmids.

All manipulations, including plasmid isolation, restriction endonuclease digestion, agarose gel electrophoresis, fragment isolation, phosphatase treatment, use of linkers, ligation, and bacterial transformations were performed by standard published procedures (Maniatis, T., E.F. Fritsch, and J. Sambrook (1982) Molecular Cloning, A Laboratory Manual).

### 11. Metabolic Labeling.

CED cells were grown for 24 hr to a density of 10⁶ cells per 6cm plate and then infected with FPV at an MOI of 10 for 120 min at 37°C. The cells were labeled with either [³H] glucosamine or [³⁵S] methionine. When [³⁵S] methionine was used, the labeling medium consisted of 9.5 ml of methioninefree DME, 4% FCS, 2 mM L-glutamine, 100 µl DME, 100 µCi [³⁵S] methionine (New England Nuclear) and carrier methionine (0.3mg/100ml). When cells were labeled with [³H] glucosamine, the DME-4% FCS lacked leucine and was supplemented with 100 µCi [³H] glucosamine (New England Nuclear) and leucine (1.46mg/100ml). Cells were harvested after approximately 40 hr, washed twice with PBS and lysed by sonicating 3 times for 5 seconds, each time in 1 ml of immunoprecipitation buffer (IPM: 10 mM Tris-HCl, pH7.2, 650 mM NaCl, 1% Triton X-100, 1% sodium deoxycholate, 0.1% SDS, 5 mM EDTA, 1 mM phenylmethylsulfonylfluoride (PMSF) and O.lmg/ml trypsin inhibitor), followed by centrifugation at 4000 RPM at 4° for 10 min. Lysates were stored at -80°.

### 12. Immunoprecipitation.

Immuniprecipitations were carried out on cell lysate samples each labeled with 1µCi [³⁵S] methionine in 0.2 ml of IPB or 1µCi [³H] glucosamine in 0.25 ml of IPB. All incubations with antibodies were done with rocking at 4°C. Antiserum was added to the cell lysate and rotated at 4° for 2 hours or overnight. 50 µl of a 1:1 solution (v/v) of Protein A-sepharose in IPB was added to each sample. Samples were rotated for one additional hour at 4°C. Samples were washed four times with 1.0 ml of IPB at 4°C, centrifuging at 12,000 RPM for 15 seconds to pellet sepharose after each wash. Pellets were washed once with 1.0 ml of TBS (TBS: 10mM Tris, 150 mM NaCl, pH8.2) at 4°C. The washes were very important to reduce background from non-specific binding. The sepharose pellets were dried by inverting them over paper towels and allowing remaining liquid to run off. Pellets were resuspended in 20 µ1 of SDS gel sample buffer (Laemmli, (1970), Nature 227:680). The samples were vortexed vigorously and heated at 100°C for 5 minutes. Samples were loaded on an SDS polyacrylamide gel which contained a 7% separation gel and a 3% stacking gel. The SDS polyacrylamide gel electrophoresis was carried out under reducing conditions and was followed by autoradiography.

### Examples

### Example 1: Definition of Promoters Active During the FPV Infectious Cycle.

The identification of pox viral DNA sequences able to control transcription in infected cells was accomplished through the use of a FPV-based transient expression assay developed for these purposes. In this system, DNA is transfected onto infected cells and, in the presence of the proper regulatory sequences, is expressed by the transcriptional apparatus of the infecting FPV. This system has been optimized for sensitivity with respect to cell type, infecting moi, the amount of exogenous DNA, the method for transfection, and time of infection, as well as incubation with the chromogenic indicator, ONPG (data not shown).

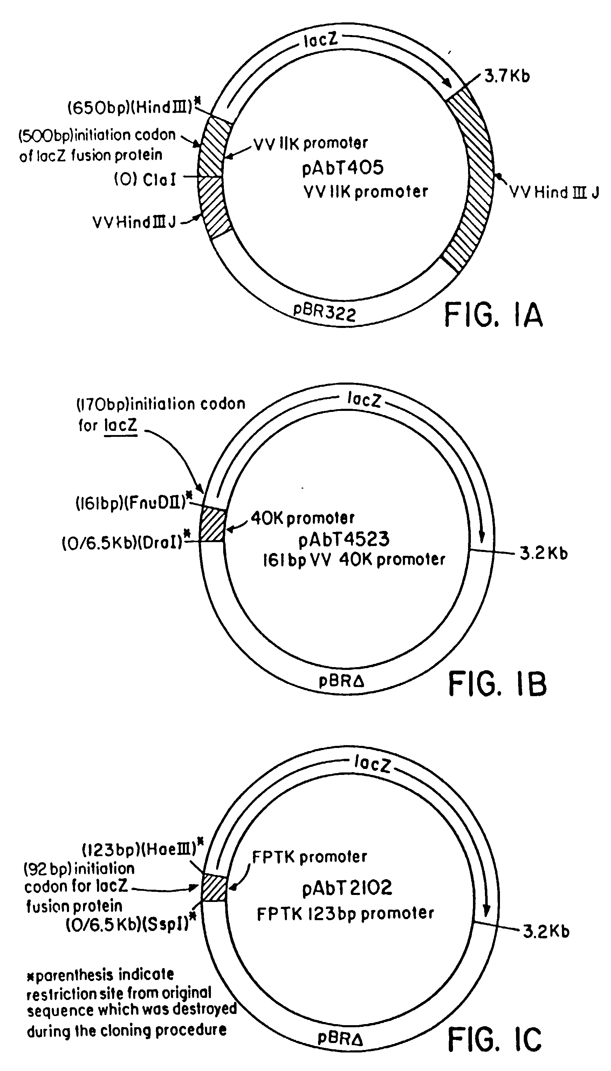

As shown in Figures 1A-1C, two vaccinia virus promoters and a presumptive FPV promoter were placed directly upstream of lacZ to allow their activities to be measured during FPV infection.

Plasmid pAbT 405 contains the 650 bp ClaI to HindIII fragment from the right-hand end of the HindIII F fragment of the vaccinia virus genome. This segment contains the vaccinia virus promoter, translational start codon, and approximately 150 base pairs from the 5'-terminus of the gene which encodes an 11 Kd polypeptide expressed after DNA replication in the infectious cycle (Bertholet, C., Drillien, R. and R. Wittek (1985) Proc. Natl. Acad. Sci. USA 82:2096-2100), ligated to the lacZ gene derived from plasmid pDP 502 (Panicali et al., (1986) Gene 193-199).

Plasmid pAbT 4523 contains the 161 bp DraI to FnuDII fragment derived from the right-hand end of the HindIII H fragment of the vaccinia virus genome ligated to the lacZ derived from plasmid pDP 500 (Panicali, D.L., A. Grzelecki, and C. Huang (1986) Gene 193-199). This lacZ gene is complete, containing its own translational start and the resulting polypeptide is, therefore, not a fusion protein. This vaccinia virus promoter element, which controls the synthesis of a 40 Kd polypeptide, functions prior to, as well as following, viral DNA replication and is classified as a constitutive promoter (Rosel, J.L., P.L. Earl, J.P. Wier, and B. Moss, (1986) J. Virol. 60:436-449).

Plasmid pAbT 2102 contains a 123 bp fragment derived from FPV genomic DNA which includes 92 bp preceding the translational start of the FPV thymidine kinase gene, the translational start codon and 29 bp of coding sequences which immediately follow (Boyle, D.B., B.H. Coupar, A.J. Gibbs, L.J. Seigman, and G.W. Both, (1987) Virology 156:355-365). Based upon the location of promoters in other poxviruses, this DNA sequence was presumed to contain the FPV promoter element and was ligated to the lacZ gene derived from plasmid pDP 503. (Panicali et al., (1986) Gene 47:193-199). The polypeptide synthesized from this construction would be a fusion of 10 amino acids derived from the FPV thymidine kinase to the beta-galactosidase polypeptide.

As shown in Table 1, each of these promoter-lacZ constructions produced measurable beta-galactosidase in the FPV-based transient expression assay, and the production of beta-galactosidase was dependent upon the appropriate plasmid. It is important to note that the assays of plasmids containing the two vaccinia virus derived promoters were read after 1.75 hr of incubation with the chromogenic substrate, while the assays containing the FPV derived promoter or no DNA were read after 9.0 hr of incubation. Clearly, each of these promoter elements are active during the FPV infectious cycle, and assuming that the measure of beta-galactosidase activity is directly proportional to promoter activity, can be ordered in decreasing activity as vaccinia 40K > vaccinia 11K > FPV TK.

**Table 1**

| DETECTION OF BETA-GALACTOSIDASE ACTIVITY IN A FPV-BASED TRANSIENT EXPRESSION ASSAY. | | |
|---|---|---|
| PLASMID | Promoter Element | A₄₂₀ |
| pAbT 405 | vaccinia virus 11K | .268* |
| pAbT 4523 | vaccinia virus 40K | .490* |
| pAbT 2102 | fowl pox virus TK | .193 |
| none | | .082 |

Confluent monolayers of 2 x 10⁵ CEF cells were infected and transfected as described in the Materials and Methods Section. A₄₂₀ was measured after 1.75 hour for those readings marked with an '*', and 9.0 hours for the remaining readings.

### Example 2: Formation and Identification of Recombinant FPV

The vaccinia virus promoter-lacZ cassette derived from pAbT 4523 was the most active in transient expression assays (Table 1). To create plasmids to facilitate the formation and identification of recombinant FPV, this BamHI-ended cassette was cloned: (1) into the XbaI site in the middle of the FPV thymidine kinase gene (pAbT 2122); (2) into the single BglII site located 960 bp from the end of the 6.8 kb BamHI J fragment of the FPV genome (pAbT 2300); and (3) in conjunction with a 702 bp DNA fragment containing the vaccinia thymidine kinase gene into the XbaI site of the FPV thymidine kinase gene (pAbT 2124).

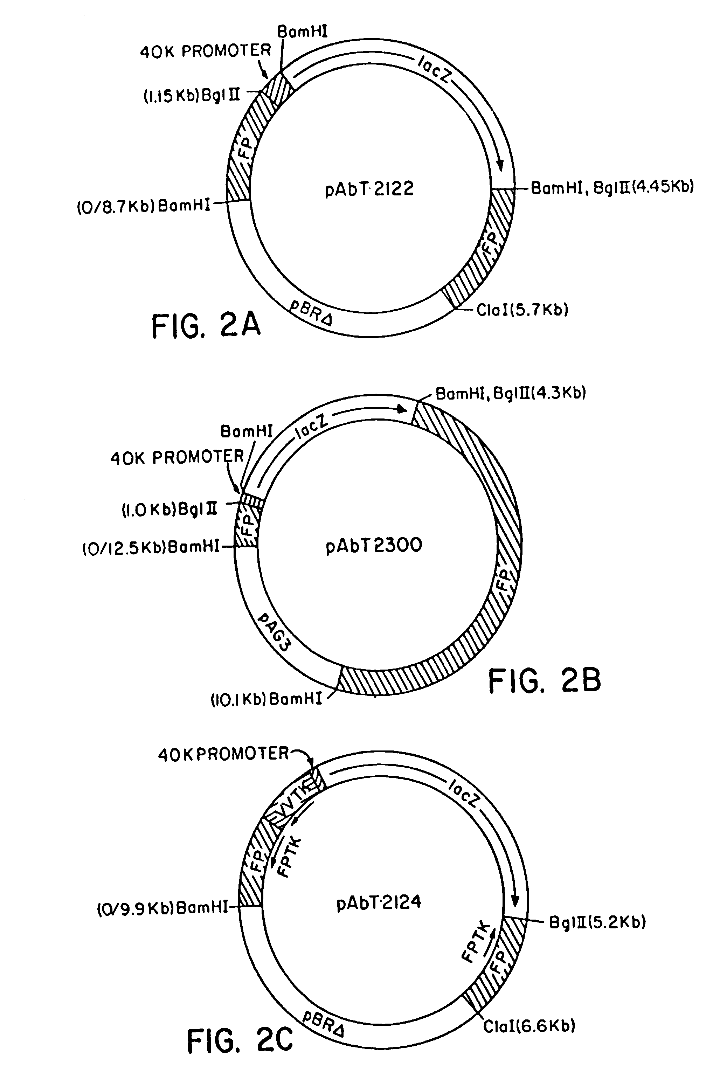

The resulting plasmids which are shown in Figures 2A-2C contain the promoter- lacZ cassette to allow the detection of recombinant virus, and flanking FPV sequences to direct insertion into the genome by homologous recombination. The FPV thymidine kinase gene was chosen for insertion as it is one of the few which have been identified on the FPV genome. For vaccinia virus it serves as an insertion site which is nonessential for growth in tissue culture (Panicali, D., and E. Paoletti (1982) Proc. Natl. Acad. Sci USA 79:4927-4931). The vaccinia virus thymidine kinase gene was included along with the promoter-lacZ cassette at this site in pAbT 2124 to complement the loss of the FPV thymidine kinase activity in case this function is is required for viral replication. Insertion of the lacZ cassette at the BglII site in the BamHI J clone was picked at random.

The conditions chosen for the formation of FPV recombinants by homologous recombination were based on those optimized for vaccinia virus and modified based on some knowledge of the growth cycle of FPV in tissue culture cells. For the initial experiment, 20 µg of pAbT 2122 were added to individual monolayers which had been infected with FPV at an moi of 0.1 at 2, 4, 6, 12, and 24 hours post-infection. Viral replication and recombination were allowed to proceed for a total of 72 hours at which time the viral progeny were harvested and the viral titers determined.

The progeny from these experiments initially were examined for the presence of recombinants with a single cell beta-galactosidase assay which allows large numbers of events to be screened. Microscopic examination revealed individual beta-galactosidase expressing cells in monolayers infected with the viral stocks in which pAbT 2122 had been employed in the transfections. No beta-galactosidase expressing cells were detected in uninfected monolayers or in monolayers which had been infected with viral stocks created in the absence of transfected DNA. These results clearly indicated that viral recombinants had been formed.

In order to confirm these results and to provide some measure of the recombination frequency, progeny from each of the viral stocks created in the presence of pAbT 2122 were allowed to form plaques and overlayed with X-gal to detect beta-galactosidase expressing virus. As shown in Table 2, a maximum recombination frequency of 0.03% was obtained by the addition of DNA 4 hours post-infection and the frequency declined when the DNA was added at later times.

**Table 2**

| FREQUENCY OF RECOMBINATION BETWEEN FPV AND A PLASMID CONTAINING THE FPV-TK INTERRUPTED BY LACZ | |
|---|---|
| Time of DNA Addn. | % Recombinants |
| 2 hr | .013 |
| 4 hr | .030 |
| 6 hr | .025 |
| 12 hr | .017 |
| 24 hr | .005 |

An average of 140,000 plaques were screened for each time point.

### Example 3: Purification of Recombinant FPV and Definition of Insertion Sites.

The experiments described above have established conditions which allow the formation of FPV recombinants. Insertion of foreign DNA into the FPV genome by homologous recombination can result in either stable gene replacements or unstable gene duplications (Spyropoulos, D.D., B.E. Roberts, D.L. Panicali, and L.K. Cohen (1988) J. Virol. 62:1046-1054). The definition of insertion sites which are nonessential for growth in tissue culture can, therefore, be only ascertained by the creation of homogeneous, stable viral stocks followed by a confirmation of the genomic structure.

To define nonessential insertion sites, plasmids pAbT 2122, 2300, and 2124 were employed in in vivo recombination (IVR) experiments as described above, and the resulting virus allowed to form plaques and overlaid with X-gal to identify recombinants. Two recombinant progeny derived from each of these individual IVRs were picked and replated to assess their purity. This process of picking and replating was repeated for 6 rounds as detailed in Table 3. The progeny derived from IVRs conducted with plasmids pAbT 2300 and 2124 were purified to produce homogeneous, stable stocks of recombinant virus, while those derived from plasmid pAbT 2122 failed to purify.

**TABLE 3**

| PURIFICATION OF RECOMBINANT VIRUS | | | | | | |
|---|---|---|---|---|---|---|
| Purification Rounds | | | | | | |
| Plasmid | 1 | 2 | 3 | 4 | 5 | 6 |
| 2300 | .002% | 11% | 53% | 94% | 90% | 100% |
| 2124 | .03% | 7% | 1% | 19% | 100% | 100% |
| 2122 | .002% | 3% | 18% | 0% | - | - |

The % recombinants formed in recombination experiments (Round 1), and their purification in subsequent rounds of plaque purification.

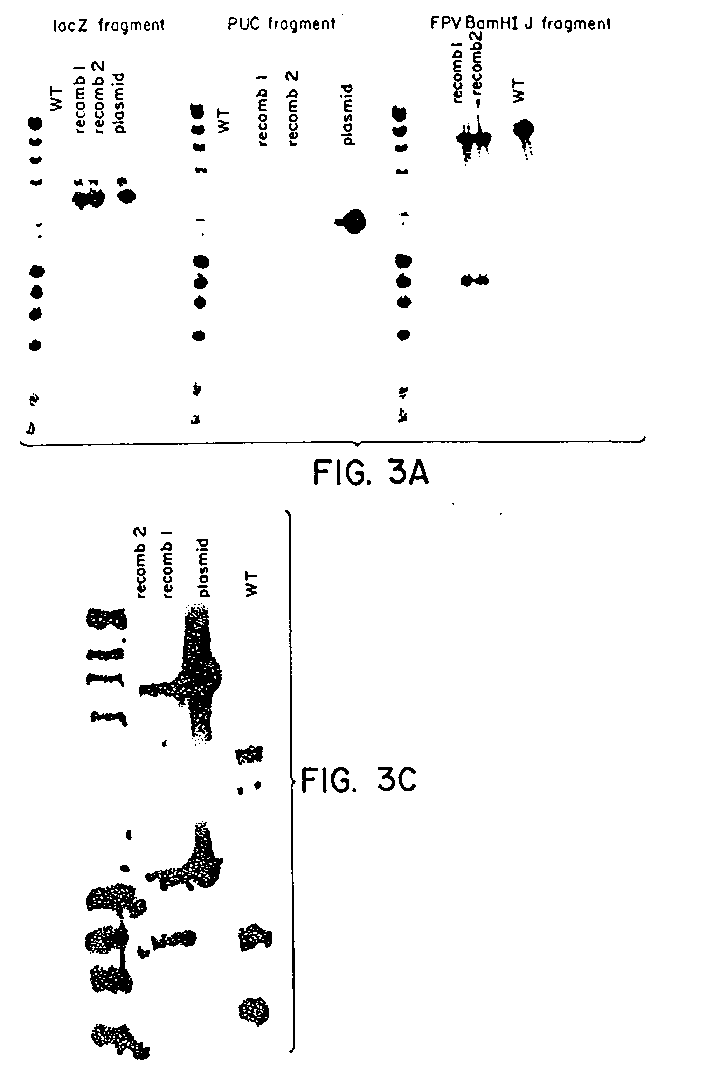

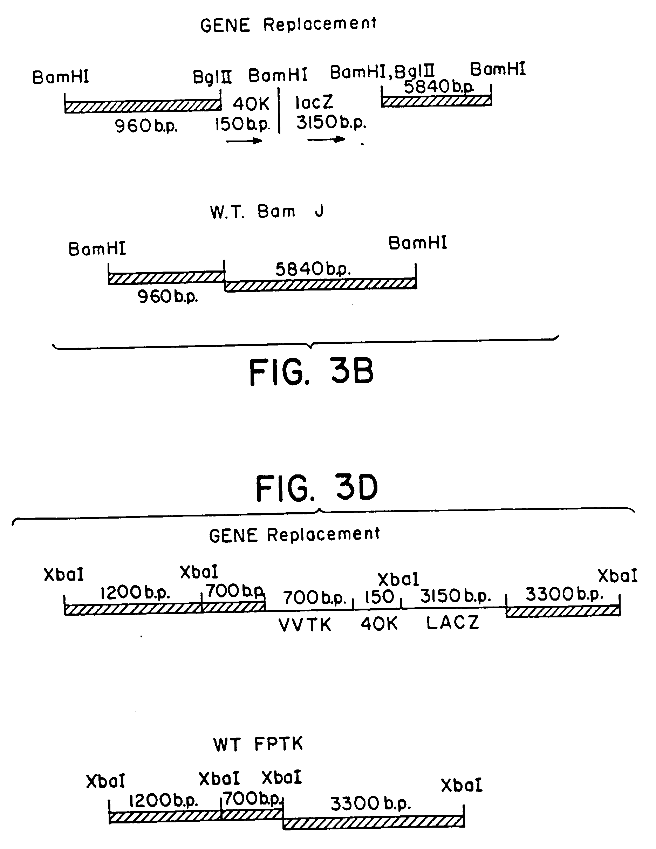

To confirm these phenotypic data, the viral progeny contained within the plaques which demonstrated homogeneity were amplified and used to prepare viral genomic DNA. Viral DNAs were digested with restriction endonucleases, resolved by agarose gel electrophoresis, and subjected to hybridization analysis with the appropriate DNA fragment. The results of an analysis of the recombinants generated with plasmid pAbT 2300 are shown in Figure 3A. Viral DNA derived from wild type FPV and two recombinant virus were digested with BamHI and hybridized to: (1) the FPV BamHI J fragment; (2) the lacZ fragment; and (3) the pUC 18-derived vector sequences. As expected, hybridization of the wild type genomic DNA with the BamHI J sequences produced a band at 6.8 kb. No hybridization was detected with either the lacZ or vector sequences. Each of the two recombinants produced identical patterns of hybridization with each of the fragments. As predicted for a recombinant FPV formed by a gene replacement event, hybridization with the BamHI J sequences produced two hybridizing species at approximately 5.8 and 1.0 kb. Hybridization with the lacZ sequences produced a single hybridizing species at 3.2 kb, and no hybridization was detected with the vector DNA. These results confirm the predicted genomic structures shown in Figure 3B, and define the BglII site in the FPV BamHI J fragment as nonessential for growth in tissue culture.

To study the effect of this insertion on the pathogenicity, immunogenicity and transmissibility of recombinant FPV in chickens, the recombinant derived from pAbT2300, designated 12-I, was inoculated into chickens. Chickens were immunized with FPV 12-I or the parental FPV via wing web stick at one day or fourteen days of age. Unimmunized chickens were used as contact controls. Sera and tissue samples were collected at 4, 10, 21 or 28 days post-immunization. Serum titers were determined by ELISA. No differences between the parental FPV or recombinant 12-I were observed with respect to pathogenicity, immunogenicity or transmissibility (data not shown).

Southern analysis was conducted on the recombinants formed with plasmid pAbT 2124. The viral DNAs derived from wild type FPV and two recombinants were digested with restriction endonuclease XbaI, and the resulting fragments resolved by agarose gel electrophoresis and hybridized to a 2.4 kb BamHI-ClaI fragment containing the FPV-TK gene. As expected, hybridization of the wild type genomic DNA produced bands at 3.3 kb, 1.2 kb, and 0.7 kb. As predicted for the recombinants formed by gene replacement events, hybridization with the FPV-TK fragment produced three species at 6.4 kb, 1.6 kb, and 1.2 kb, as shown in Figure 3C. These results confirm the predicted genomic structures shown in Figure 3D, and indicate that the FPV-TK gene can be used as an insertion site when complemented by the vaccinia virus TK.

### Example 4: FPV-Derived Promoter Elements

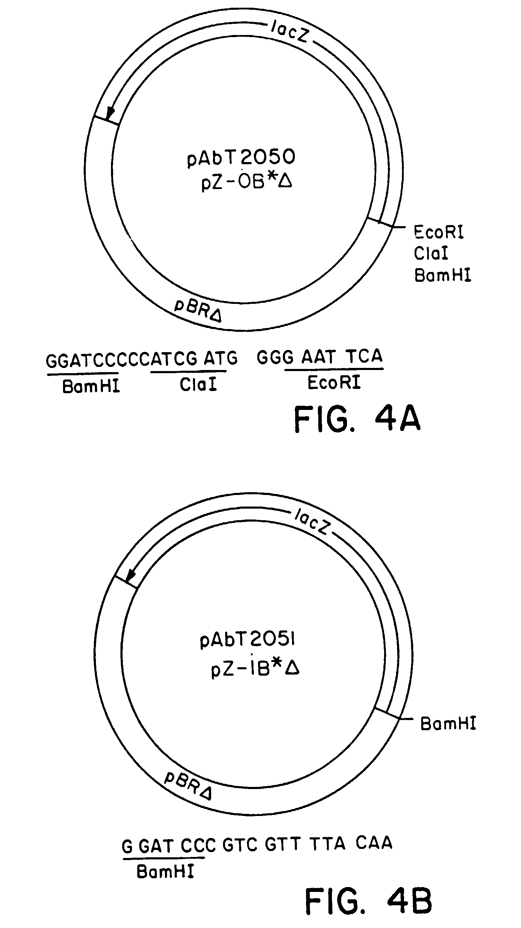

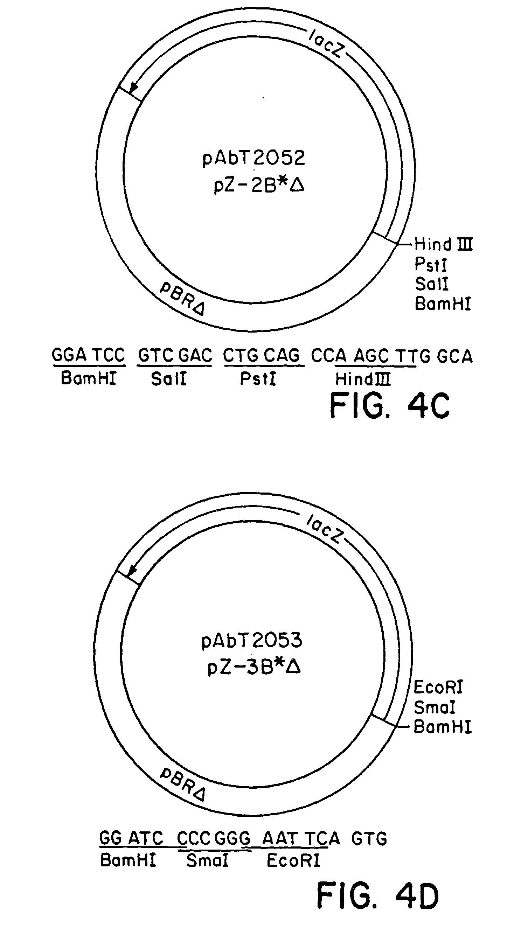

The promoter used in the identification of the recombinant FPV as well as the two most active promoters detected in the transient expression assays were obtained from vaccinia virus genomic DNA. In order to derive a eukaryotic expression system based entirely upon FPV an attempt was made to define FPV-derived elements as active as the two vaccinia promoters. As shown in Figure 4, plasmids pAbT 2050, 2051, 2052 and 2053 contain the lacZ gene preceded by a single BamHI insertion site (Panicali, D.L., A. Grzelecki, and C. Huang (1986) Gene 47:193-199). The plasmids differ from each other in that the lacZ gene in pAbT 2050 contains its own translational start codon while the lacZ gene in the other three plasmids do not contain start codons but are positioned in each of the three possible reading frames with respect to the BamHI site. Fragments cloned into all four plasmids can be assayed for their ability to direct transcription of lacZ, producing beta-galactosidase in transient expression assays. This allows the identification of fragments which contain isolated promoter elements with or without their associated translational start codons (Panicali, D.L., A. Grzelecki, and C. Huang, (1986) Gene 47:193-199).

The fragments derived from a total Sau 3AI digest of FPV genomic DNA were cloned into the single BamHI site in each of the four plasmids shown in Figures 4A-4D. The average size of the Sau 3AI fragments was approximately 150-200 bp. Initially, the resulting clones were screened for promoter activity in E. coli as previous studies have demonstrated the functional cross-reactivity of poxvirus and prokaryotic promoters (data not shown). Plasmid DNA was isolated from those bacterial colonies which expressed beta-galactosidase and tested for authentic promoter activity in the FPV-based transient expression assay.

Table 4 shows the results from a transient expression assay conducted on 16 pools, each containing 5 of the Sau 3AI clones. Clearly, beta-galactosidase activity is detectable in assays conducted with the DNAs present in pool C. To assess the individual contributions to that activity, the individual clones which comprise pool C were separately tested. As seen in Table 5, only four of the five clones composing group C could be revived from frozen stocks, but two of the clones, designated C₁ and C₂, demonstrated significant promoter activity.

**Table 4**

| IDENTIFICATION OF FPV-DERIVED PROMOTERS IN THE FPV-BASED TRANSIENT EXPRESSION ASSAY | |
|---|---|
| DNA POOL | A₄₂₀ |
| B | .059 |
| C | .128 |
| D | .065 |
| E | .056 |
| F | .053 |
| G | .056 |
| H | .056 |
| I | .063 |
| J | .058 |
| K | .067 |
| L | .057 |
| M | .058 |
| N | .054 |
| P | .063 |
| Q | .067 |
| S | .069 |
| none | .055 |

**Table 5**

| FOWLPOX PROMOTER ACTIVITY OF POOL C CLONES | |
|---|---|
| DNA POOL | A₄₂₀ |
| C₁ | .507 |
| C₂ | .119 |
| C₃ | .071 |
| C₅ | .063 |
| none | .057 |

A further characterization of FPV promoters C₁ and C₂ including a comparison of their strengths with those of the two previously characterized vaccinia virus promoters is shown in Table 6. This set of transient expression assays was conducted in the presence and absence of cytosine arabinoside (araC). As an inhibitor of viral DNA replication, ara C allows the temporal regulation of each of the promoters to be defined. Late promoters such as the 11K vaccinia virus promoter in pAbT 405 are dependent upon DNA replication for expression and are, therefore, essentially inactive in the presence of araC. Early promoters which express prior to DNA replication are relatively unaffected by the inclusion of araC. Constitutive promoters such as the 40K promoter in pAbT 4523 express both prior to and following DNA replication. These show some diminution of activity in the presence of araC. The results for the FPV promoters C₁ and C₂ suggest that both are early promoters. The possibility that they possess some late activity and in fact are constituitive promoters cannot be eliminated from these data. It is significant that the FPV promoter C₁ demonstrates activity at least equivalent in strength to the strongest vaccinia virus promoter (pAbT 4523).

**Table 6**

| COMPARISON OF FOWLPOX PROMOTERS C₁ and C₂ WITH VACCINIA PROMOTERS | | |
|---|---|---|
| DNA | AraC | A₄₂₀ |
| pAbT 405 | - | .380 |
| pAbT 405 | + | .034 |
| pAbT 4523 | - | .853 |
| pAbT 4523 | + | .563 |
| C₁ | - | 1.656 |
| C₁ | + | 1.523 |
| C₂ | - | .133 |
| C₂ | + | .103 |
| none | - | .019 |
| none | + | .026 |

Each of the two FPV promoters has been sequenced and these results are shown in Figures 5A and 5B.

Promoter C₁ contains its own translational start codon and is linked to the lacZ by a coding fusion. To allow the use of this promoter with genes which contain their own translational start codons, two modifications have been made which are also shown in Figure 5C. In the first the ATG has been changed to an ATA and in the second a termination codon, TAA has been placed 6 nucleotides downstream from the endogenous translational start. These modifications were achieved through the chemical synthesis of two separate 38 bp Dra I to BamHI DNA linkers and the replacement by excision and religation of the DraI to Sau 3AI fragment of the original C₁ promoter with these modified sequences. Each of the modified promoters were ligated to a lacZ which contained its own translational start codon, and compared to the original promoter in transient expression assays. As shown in Table 7 both modifications successfully eliminated the translational start while retaining promoter activity.

**TABLE 7**

| ACTIVITY OF MODIFIED C₁ FPV PROMOTER IN TRANSIENT EXPRESSION ASSAYS. | |
|---|---|
| Promoter | A₄₂₀ |
| C₁ | .341 |
| 2137 | .616 |
| 2138 | .331 |

Transient expression assays were read after 3.0 hrs of incubation in the presence of ONPG. Plasmid pAbT 2137 contains the ATG to ATA modification, and pAbT 2138 contains the TAA insertion.

Promoter C₂ contains no translational signals and is used directedly as cloned.

### Example 5: Generalized Fowlpox Expression Vectors.

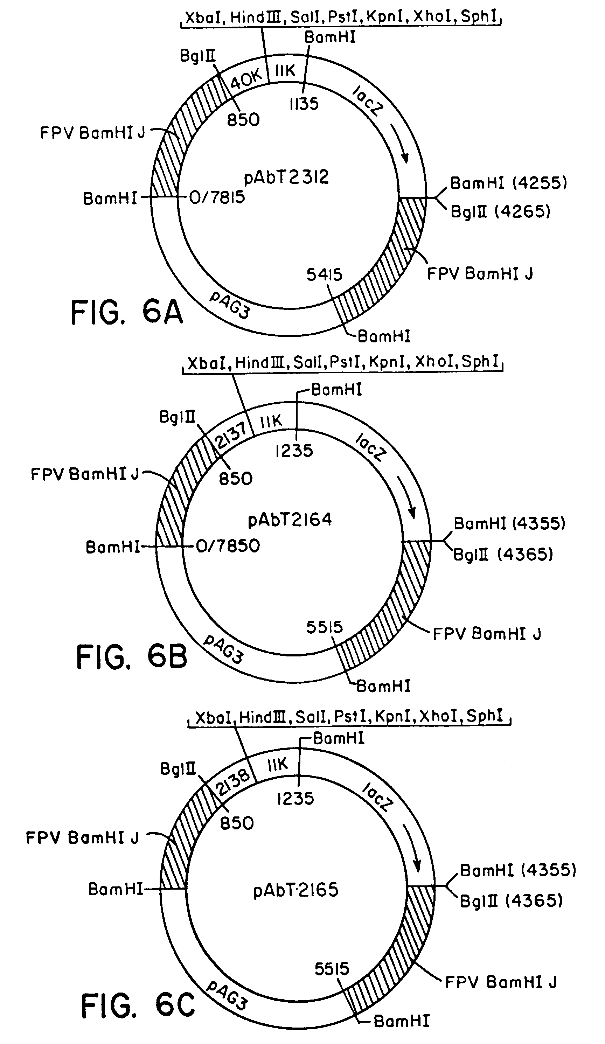

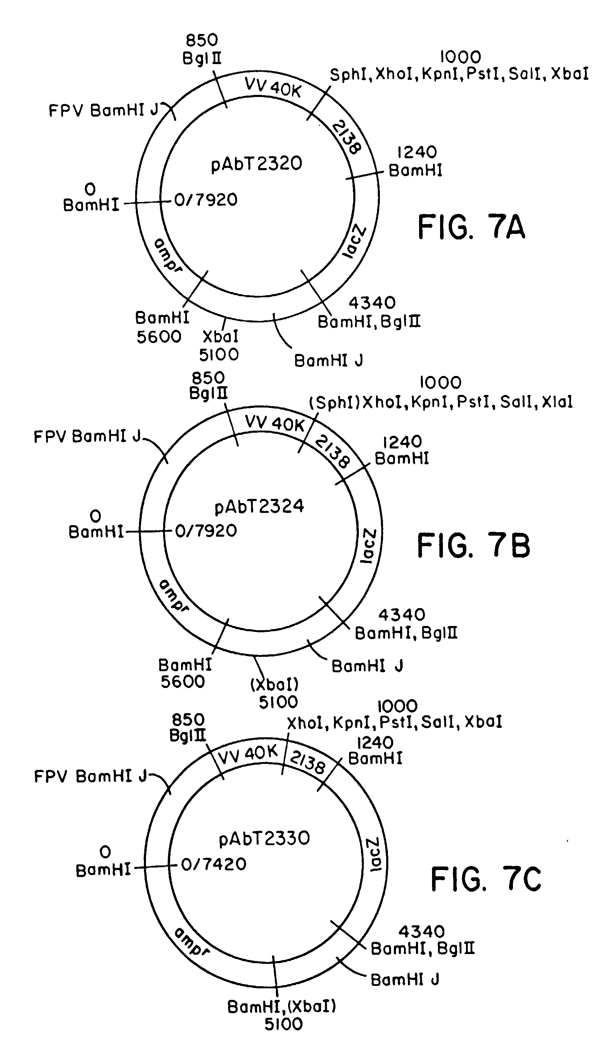

In order to create a system for the expression of any foreign DNA sequences in recombinant FPV, a series of plasmids have been created which are shown in Figures 6 and 7. In addition to DNA sequences which allow selection and replication of these plasmids in bacterial hosts, each plasmid also contains: (1) FPV-derived flanking sequences to direct insertion to a site nonessential for replication in tissue culture; (2) a FPV-active promoter followed by a DNA sequence which contains a multiple cloning site for the insertion of the foreign DNA to be expressed; and (3) an additional FPV-active promoter regulating the synthesis of the lacZ to allow the rapid identification of recombinant virus.

Plasmids pAbT 2312, 2164, and 2165 (Figure 6) are designed to facilitate the formation of viral recombinants at the BglII site of the FPV BamHI J fragments. They differ in whether vaccinia virus or FPV promoter elements are employed. Plasmid pAbT 2312 uses the vaccinia promoters with the stronger promoter derived from plasmid pAbT 4523 (40K promoter) employed to control the synthesis of the foreign gene and the weaker promoter derived from pAbT 405 (11K promoter) to control the synthesis of the lacZ. Plasmids pAbT 2164 and 2165 employ the FPV 2137 and 2138 promoters, respectively, to control the synthesis of the foreign gene and the vaccinia 11K to control the synthesis of the lacZ.

Plasmids pAbT 2320, 2324 and 2330 (Figure 7) are vectors designed to facilitate the formation of viral recombinants at the BglII site of the FPV BamHI J fragment. These three plasmids each contain the vaccinia virus 40K promoter followed by a multiple cloning site for the insertion of foreign genes, and the FPV 2138 promoter controlling the E. coli lacZ gene for selection of recombinant FPV, inserted into the Bg1II site within the 6.8kb FPV BamHI J fragment for insertion of the foreign gene into the FPV genome.

pAbT 2324 differs from pAbT 2320 in that the SphI site in the multiple cloning site and the XbaI site in the BamHI J fragment have been removed by treatment of each cleavage site with Klenow and subsequent religation of the plasmid. pAbT 2330 differs from pAbT 2324 in that the DNA sequences designated 5100 through 5600 corresponding to a XbaI-BamHI fragment from the BamHI J fragment (see Figure 7) have been removed.

### Example 6: Expression of avian infectious bronchitis virus spike protein in recombinant FPV.

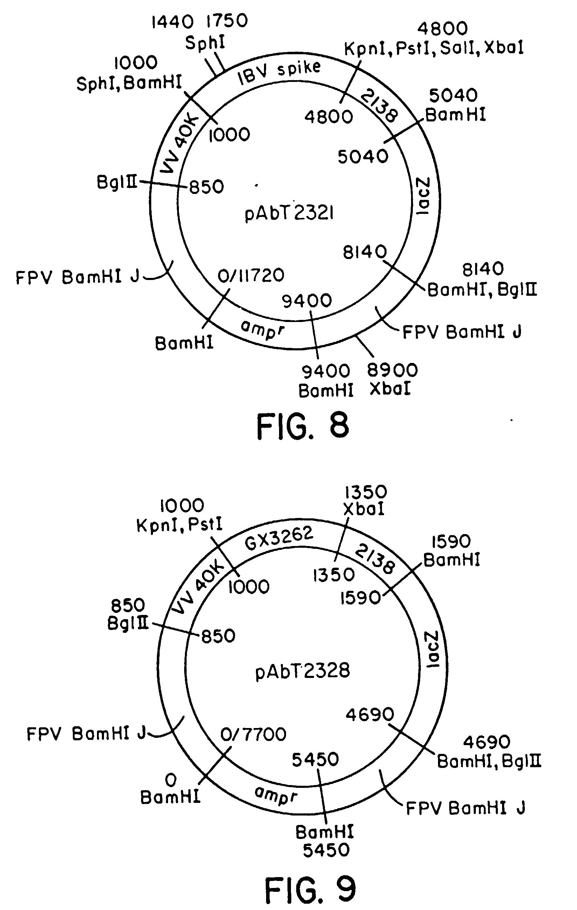

The gene encoding the spike protein of avian infectious bronchitis virus strain m41 was obtained from Paul Sondermeijer (Intervet International, Boxmeer, The Netherlands). The gene was contained on a 2800 bp XbaI fragment and was inserted into the unique XhoI site in pAbT 2320 to form pAbT 2321, as shown in Figure 8.

pAbT 2321 is a vector for the insertion and expression of avian infectious bronchitis virus spike protein in FPV. pAbT 2321 contains the spike protein gene under the control of the vaccinia virus 40K promoter, the E. coli lacZ gene under the control of the FPV 2138 promoter for selection of FPV recombinants, flanked by portions of the FPV BamHI J fragment for directing recombination into the FPV genome, and a bacterial replicon and ampicillin-resistance gene for growth and selection in E. coli.

pAbT 2321 was used as a vector to insert the spike protein gene into FPV by in vivo recombination as described in the Materials and Methods Section. Recombinants, designated FPV 59-5, were obtained and purified. Immunoprecipitation analysis using infected CED cells labeled with ³H-glucosamine and antisera against IBV (see Materials and Methods) revealed the synthesis of spike proteins of the expected sizes.

### Example 7: Insertion of Eimeria genes into recombinant FPV.

Eimeria species are the causative agents of poultry coccidiosis. Genes encoding immunogenic antigens have been identified by Miller et al. (1989) Infect. Immun. 57:2014.

One such antigen, denoted GX3262, is encoded by a 350 bp partial cDNA clone. This gene was obtained from R. Strausberg (Genex Corp., Gaithersberg, MD) and was inserted into pAbT 2330 to form pAbT 2328, as shown in Figure 9..

pAbT 2328 is a vector for the insertion and expression of GX3262 from Eimeria tenella, pAbT 2328 contains GX3262 under the control of the vaccinia virus 40K promoter, the E. coli lacZ gene under the control of the FPV 2138 promoter for selection of FPV recombinants, portions of the FPV BamHI J fragment for directing recombination into the FPV genome and a bacterial replicon and ampicillin-resistance gene for growth and selection in E. coli.

Plasmid pAbT 2328 was used as a vector to insert the Eimeria tenella gene GX3262 into FPV by in vivo recombination. Recombinants, designated FPV 71, were obtained.

### Deposits

The plasmids pAbT 2164 and pAbT 2320 were deposited at the American Type Culture Collection in Rockville, Maryland and assigned the accession numbers 40485 and , respectively.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. Recombinant fowlpox virus capable of expressing foreign DNA in a host, wherein the foreign DNA is inserted in a region of the viral genome homologous to the BamHI J fragment of the fowlpox virus, and wherein said insertion does not substantially reduce the immunogenicity of the recombinant virus in a host animal.

2. A recombinant fowlpox virus of Claim 1, wherein the foreign DNA sequence encodes an immunogenic protein of a pathogen.

3. A recombinant fowlpox virus of Claim 2, wherein the immunogenic protein is derived from a fowl pathogen.

4. A recombinant fowlpox virus of Claim 1, wherein the foreign DNA is controlled by a fowlpox viral promoter.

5. A recombinant fowlpox virus of Claim 4, wherein the fowlpox viral promoter is selected from the group consisting of the C1, C2 promoters and modified versions thereof having the DNA sequences shown in Figure 5.

6. A recombinant fowlpox virus of Claim 3, wherein the pathogen is a parasite of species Eimeria.

7. A recombinant fowlpox virus of Claim 6, wherein the immunogenic protein is Eimeria tenella CX3262.

8. A DNA vector for in vivo recombination with a fowlpox virus designed to produce a recombinant fowlpox virus capable of expressing at least one foreign DNA sequence and retaining its immunogenicity, comprising:
a) at least one pox viral promoter DNA sequence linked to;
b) at least one DNA sequence containing a multiple cloning site for insertion of foreign DNA, each cloning site positioned such that expression of each inserted foreign DNA will be controlled by an individual pox promoter of element (a); and
c) fowlpox DNA sequences flanking elements (a) and (b) at both the 5' and 3' ends wherein the flanking sequences are homologous to the BamHI J fragment of the fowlpox genome, or a portion thereof sufficient for recombination.

9. A DNA vector of Claim 8, wherein the pox promoter is a fowlpox promoter selected from the group consisting of C1 and C2 promoters, or modified versions thereof, having the DNA sequence shown in Figure 5.

10. A DNA vector of Claim 9, wherein the 5' flanking sequences are homologous to DNA sequences of the BamHI J fragment 5' of the Bg1II site and the 3' flanking sequences are homologus to DNA sequences of the BamHI J fragment 3' of the Bg1II site.

11. A DNA vector of Claim 8, further comprising:
d) a pox viral promoter linked to a gene which encodes a marker or indicator for selection of recombinant fowlpox virus, the promoter and gene located between the flanking DNA sequences of the DNA vector;
e) a replicon for vector replication in a prokaryotic host; and
f) a gene encoding a selectable marker for selection of the rector in a prokaryotic host, the gene being under control of a host cell promoter.

12. A DNA vector of Claim 11, wherein the marker gene used for selection of recombinant virus is the lacZ gene.

13. A vector for insertion of foreign DNA into a fowlpox virus to produce a recombinant fowlpox virus containing the foreign DNA, comprising:
a) a promoter linked to;
b) a foreign DNA sequence;
c) a second promoter linked to a gene encoding a marker or indicator for selection of recombinant fowlpox virus;
d) DNA sequences of fowlpox virus flanking 5' and 3' to the construct of elements a-c, the flanking fowlpox viral DNA sequences being homologous to a region of the viral genome where elements a-c are to be inserted, wherein the flanking sequences are homologous to the BamHI J fragment of the fowlpox genome, or a portion thereof sufficient for recombination, the region being one in which insertion of foreign DNA does not substantially reduce the immunogenicity of the recombinant virus in a host animal;
e) a replicon for vector replication in a prokaryotic host cell; and
f) a structural gene encoding a marker or indicator for selection of the vector in a prokaryotic host, the gene being under control of a prokaryotic promoter.

14. A vector of Claim 13, wherein the promoters are derived from fowlpox virus.

15. A vector of Claim 14, wherein the fowlpox promoter is selected from the group consisting of the C1 and C2 promoters, or modified versions thereof, having the DNA sequence shown in Figure 5.

16. A vector of Claim 13, wherein the foreign DNA sequence is a sequence encoding an immunogenic protein or protein fragment of a pathogen.

17. A vector of Claim 16, wherein the pathogen is a parasite of species Eimeria.

18. A vector of Claim 17, wherein the immunogenic protein is Eimeria tenella Gx3262.

19. A vector of Claim 13, wherein the structural or marker gene under control of the second promoter is the lacZ gene.

20. A DNA vector of Claim 13, wherein the 5' flanking sequences are homologous to DNA sequences of the BamHI J fragment 5' of the Bg1II site and the 3' flanking sequences are homologous to DNA sequences of the BamHI J fragment 3' of the Bg1II site.

21. A recombinant, immunologically intact, fowlpox virus produced by:
a) inserting one or more foreign DNA sequences into a vector of Claim 8; and
b) allowing the vector containing foreign DNA to undergo in vivo recombination with fowlpox virus to produce a recombinant fowlpox virus having inserted into its genome the foreign DNA sequences and a fowlpox promoter capable of controlling the expression of these foreign DNA sequences.

22. A recombinant fowlpox virus of Claim 21, wherein the pox viral promoter is a fowlpox promoter selected from the group consisting of the C1 and C2 promoter, or modified versions thereof, having the DNA sequences shown in Figure 5.

23. A recombinant fowlpox virus of Claim 21, wherein the foreign DNA sequences are those encoding one or more immunogenic proteins or protein fragments of pathogen.

24. A recombinant fowlpox virus of Claim 23, wherein the pathogen is a parasite of species Eimeria.

25. A recombinant fowlpox virus of Claim 24, wherein the immunogenic protein is Eimeria tenella Gx3262.

26. Use of a recombinant fowlpox virus capable of expressing foreign DNA encoding one or more immunogenic proteins of a pathogen, wherein the foreign DNA is inserted in a region of the viral genome, homologous to the BamHI J fragment of the fowlpox virus, insertion of which does not substantially reduce the immunogenicity of the recombinant fowlpox in a host animal, expression of each immunogenic protein being under the control of a fowlpox promoter, for the manufacture of a vaccine for vaccinating poultry or other animal against the pathogen.

27. The use of Claim 26, wherein the pathogen is a parasite of species Eimeria.

28. The use of Claim 26, wherein the immunogenic protein is Eimeria tenella GX3262.

## Patentansprüche

1. Rekombinantes Geflügelpockenvirus, das Fremd-DNA in einem Wirt exprimieren kann, worin die Fremd-DNA in einen Bereich des viralen Genoms inseriert wird, der zu dem BamHI J Fragment des Geflügelpockenvirus homolog ist, und worin die besagte Insertion die Immunogenität des rekombinanten Virus in einem Wirtstier nicht wesentlich reduziert.

2. Rekombinantes Geflügelpockenvirus nach Anspruch 1, worin die Fremd-DNA-Sequenz ein immunogenes Protein eines Erregers kodiert.

3. Rekombinantes Geflügelpockenvirus nach Anspruch 2, worin das immunogene Protein von einem Geflügelkrankheitserreger abgeleitet ist.

4. Rekombinantes Geflügelpockenvirus nach Anspruch 1, worin die Fremd-DNA von einem viralen Geflügelpockenpromotor gesteuert wird.

5. Rekombinantes Geflügelpockenvirus nach Anspruch 4, worin der virale Geflügelpockenpromotor ausgewählt ist aus der Gruppe bestehend aus den C1, C2 Promotoren und deren modifizierten Versionen, die die in Abbildung 5 gezeigte DNA-Sequenz aufweisen.

6. Rekombinantes Geflügelpockenvirus nach Anspruch 3, worin der Erreger ein Parasit der Spezies Eimeria ist.

7. Rekombinantes Geflügelpockenvirus nach Anspruch 6, worin das immunogene Protein Eimeria tenella GX3262 ist.

8. DNA-Vektor für die in vivo Rekombination mit einem Geflügelpockenvirus, der dafür vorgesehen ist, ein rekombinantes Geflügelpockenvirus herzustellen, das mindestens eine Fremd-DNA-Sequenz exprimieren und seine Immunogenität beibehalten kann, umfassend:
a) mindestens eine damit verbundene virale Pockenpromotor-DNA-Sequenz;
b) mindestens eine DNA-Sequenz enthaltend eine Mehrfachklonierungsstelle zur Insertion von Fremd-DNA, wobei jede Klonierungsstelle derart positioniert ist, daß die Expression jeder inserierten Fremd-DNA von einem einzelnen Pockenpromotor von Element (a) gesteuert wird; und
c) flankierende Elemente (a) und (b) der Geflügelpocken-DNA-Sequenzen sowohl an den 5' und 3' Enden, worin die flankierenden Sequenzen zu dem BamHI J Fragment des Geflügelpockengenoms, oder einem Teil davon, der für die Rekombination ausreicht, homolog sind.

9. DNA-Vektor nach Anspruch 8, worin der Pockenpromotor ein Geflügelpockenpromotor ist, der ausgewählt ist aus der Gruppe bestehend aus C1 und C2 Promotoren oder deren modifizierten Versionen, die die in Abbildung 5 gezeigte DNA-Sequenz aufweisen.

10. DNA-Vektor nach Anspruch 9, worin die am 5' Ende flankierenden Sequenzen zu den DNA-Sequenzen des BamHI J Fragments am 5' Ende der BglII-Stelle homolog sind, und die am 3' Ende flankierenden Sequenzen zu den DNA-Sequenzen des BamHI J Fragments am 3' Ende der BglII-Stelle homolog sind.

11. DNA-Vektor nach Anspruch 8, weiterhin umfassend:
d) einen viralen Pockenpromotor, der mit einem Gen verbunden ist, das einen Marker oder Indikator kodiert zur Auswahl des rekombinanten Geflügelpockenvirus, wobei der Promotor und das Gen zwischen den flankierenden DNA-Sequenzen des DNA-Vektors liegen;
e) ein Replikon zur Vektor-Replikation in einem prokaryontischen Wirt; und
f) ein Gen, das einen selektierbaren Marker kodiert, zur Auswahl des Vektors in einem prokaryontischen Wirt, wobei das Gen von einem Wirtszellenpromotor gesteuert wird.

12. DNA-Vektor nach Anspruch 11, worin das zur Auswahl des rekombinanten Virus verwendete Marker-Gen das lacZ-Gen ist.

13. Vektor zur Insertion von Fremd-DNA in ein Geflügelpockenvirus zur Herstellung eines rekombinanten Geflügelpockenvirus, das die Fremd-DNA enthält, umfassend:
a) einen damit verbundenen Promotor;
b) eine Fremd-DNA-Sequenz;
c) einen zweiten Promotor, der mit einem Gen verbunden ist, das einen Marker oder Indikator kodiert, zur Auswahl des rekombinanten Geflügelpockenvirus;
d) DNA-Sequenzen des Geflügelpockenvirus, die am 5' und 3' Ende das Konstrukt der Elemente a-c flankieren, wobei die flankierenden viralen Geflügelpocken-DNA-Sequenzen zu einem Bereich des viralen Genoms homolog sind, wo die Elemente a-c inseriert werden sollen, worin die flankierenden Sequenzen zu dem BamHI J Fragment des Geflügelpockengenoms, oder einem Teil davon, der für die Rekombination ausreicht, homolog sind, wobei der Bereich ein solcher ist, worin eine Insertion von Fremd-DNA die Immunogenität des rekombinanten Virus in einem Wirtstier nicht wesentlich reduziert;
e) ein Replikon zur Vektor-Replikation in einer prokaryontischen Wirtszelle; und
f) ein Strukturgen, das einen Marker oder Indikator kodiert, zur Auswahl des Vektors in einem prokaryontischen Wirt, wobei das Gen von einem prokaryontischen Promotor gesteuert wird.

14. Vektor nach Anspruch 13, worin die Promotoren von dem Geflügelpockenvirus abgeleitet sind.

15. Vektor nach Anspruch 14, worin der Geflügelpockenpromotor ausgewählt ist aus der Gruppe bestehend aus den C1 und C2 Promotoren, oder deren modifizierten Versionen, die die in Abbildung 5 gezeigte DNA-Sequenz aufweisen.

16. Vektor nach Anspruch 13, worin die Fremd-DNA-Sequenz eine Sequenz ist, die ein immunogenes Protein oder Proteinfragment eines Erregers kodiert.

17. Vektor nach Anspruch 16, worin der Erreger ein Parasit der Spezies Eimeria ist.

18. Vektor nach Anspruch 17, worin das immunogene Protein Eimeria tenella GX3262 ist.

19. Vektor nach Anspruch 13, worin das von dem zweiten Promotor gesteuerte Struktur- oder Marker-Gen das lacZ-Gen ist.

20. DNA-Vektor nach Anspruch 13, worin die am 5' Ende flankierenden Sequenzen zu den DNA-Sequenzen des BamHI J Fragments am 5' Ende der BglII-Stelle homolog sind, und die am 3' Ende flankierenden Sequenzen zu den DNA-Sequenzen des BamHI J Fragments am 3' Ende der BglII-Stelle homolog sind.

21. Rekombinantes, immunologisch intaktes Geflügelpockenvirus, das hergestellt wird, indem man:
a) eine oder mehrere Fremd-DNA-Sequenzen in einen Vektor nach Anspruch 8 inseriert; und
b) den die Fremd-DNA enthaltenden Vektor in vivo mit Geflügelpockenvirus rekombinieren läßt zur Herstellung eines rekombinanten Geflügelpockenvirus, in dessen Genom die Fremd-DNA-Sequenzen und ein Geflügelpockenpromotor inseriert sind, der die Expression dieser Fremd-DNA-Sequenzen steuern kann.

22. Rekombinantes Geflügelpockenvirus nach Anspruch 21, worin der virale Pockenpromotor ein Geflügelpockenpromotor ist, der ausgewählt ist aus der Gruppe bestehend aus dem C1 und C2 Promotor oder deren modifizierten Versionen, die die in Abbildung 5 gezeigte DNA-Sequenz aufweisen.

23. Rekombinantes Geflügelpockenvirus nach Anspruch 21, worin die Fremd-DNA-Sequenzen solche sind, die ein oder mehrere immunogene Proteine oder Proteinfragmente eines Erregers kodieren.

24. Rekombinantes Geflügelpockenvirus nach Anspruch 23, worin der Erreger ein Parasit der Spezies Eimeria ist.

25. Rekombinantes Geflügelpockenvirus nach Anspruch 24, worin das immunogene Protein Eimeria tenella GX3262 ist.

26. Verwendung eines rekombinanten Geflügelpockenvirus, das Fremd-DNA exprimieren kann, die ein oder mehr immunogene Proteine eines Erregers kodiert, worin die Fremd-DNA in einen Bereich des viralen Genoms inseriert ist, der zu dem BamHI J Fragment des Geflügelpockenvirus homolog ist, wobei die Insertion der Fremd-DNA die Immunogenität des rekombinanten Geflügelpockenvirus in einem Wirtstier nicht wesentlich reduziert, wobei die Expression jedes immunogenen Proteins von einem Geflügelpockenpromotor gesteuert wird, zur Herstellung eines Impfstoffes zum Impfen von Geflügel oder anderen Tieren gegen den Erreger.

27. Verwendung nach Anspruch 26, worin der Erreger ein Parasit der Spezies Eimeria ist.

28. Verwendung nach Anspruch 26, worin das immunogene Protein Eimeria tenella GX3262 ist.

## Revendications

1. Avipoxvirus recombinant capable d'exprimer un ADN étranger dans un hôte, dans lequel l'ADN étranger est inséré dans une région du génome viral homologue au fragment BamHI J de l'avipoxvirus et dans lequel ladite insertion ne réduit pas considérablement l'immunogénicité du virus recombinant dans un animal hôte.

2. Avipoxvirus recombinant selon la revendication 1, dans lequel la séquence d'ADN étranger code pour une protéine immunogène d'un agent pathogène.

3. Avipoxvirus recombinant selon la revendication 2, dans lequel la protéine immunogène est dérivée d'un agent pathogène aviaire.

4. Avipoxvirus recombinant selon la revendication 1, dans lequel l'ADN étranger est contrôlé par un promoteur viral de l'avipoxvirus.

5. Avipoxvirus recombinant selon la revendication 4, dans lequel le promoteur viral de l'avipoxvirus est choisi au sein du groupe constitué par les promoteurs C1, C2 et leurs versions modifiées possédant les séquences d'ADN présentées à la figure 5.

6. Avipoxvirus recombinant selon la revendication 3, dans lequel l'agent pathogène est un parasite de l'espèce Eimeria.

7. Avipoxvirus recombinant selon la revendication 6, dans lequel la protéine immunogène est la protéine GX3262 d'Eimeria tenella.

8. Vecteur d'ADN permettant une recombinaison in vivo avec l'avipoxvirus conçu de manière à produire un avipoxvirus recombinant capable d'exprimer au moins une séquence d'ADN étranger et de conserver son immunogénicité, comprenant:
a) au moins une séquence d'ADN du promoteur de virus pox liée à:
b) au moins une séquence d'ADN contenant un site de clonage multiple permettant l'insertion d'une séquence d'ADN étranger, chaque site de clonage étant positionné de telle manière que l'expression de chaque séquence d'ADN étranger insérée sera contrôlé par un promoteur pox d'un élément (a) indépendant: et
c) les séquences d'ADN de l'avipoxvirus adjacentes aux éléments (a) et (b) aux extrémités 5' et 3' dans lesquelles les séquences adjacentes sont homologues au fragment BamHI J du génome de l'avipoxvirus, ou une partie de celui-ci suffisante pour permettre la recombinaison.

9. Vecteur d'ADN selon la revendication 8 dans lequel le promoteur pox est un promoteur de l'avipoxvirus choisi au sein du groupe constitué par les promoteurs C1 et C2, ou des versions modifiées de celui-ci possédant la séquence d'ADN présentée à la figure 5.

10. Vecteur d'ADN selon la revendication 9, dans lequel les séquences adjacentes en 5' sont homologues aux séquences d'ADN du fragment BamHI J en 5' du site Bglll et les séquences adjacentes en 3' sont homologues aux séquences d'ADN du fragment BamHI J en 3' du site Bglll.

11. Vecteur d'ADN selon la revendication 8, comprenant en outre:
d) un promoteur de virus pox lié à un gène qui code pour un marqueur ou indicateur destiné à la sélection de l'avipoxvirus recombinant, le promoteur et le gène étant localisés entre les séquences d'ADN adjacentes au vecteur d'ADN;
e) un réplicon destiné à la réplication du vecteur dans un hôte procaryote; et
f) un gène codant pour un marqueur sélectionnable permettant la sélection du vecteur dans un hôte procaryote, le gène étant sous le contrôle d'un promoteur de la cellule hôte.

12. Vecteur d'ADN selon la revendication 11, dans lequel le gène marqueur utilisé pour sélectionner le virus recombinant est le gène lac Z.

13. Vecteur destiné à l'insertion d'un ADN étranger dans un avipoxvirus afin de produire un avipoxvirus recombinant contenant l'ADN étranger, comprenant:
a) un promoteur lié à:
b) une séquence d'ADN étranger;
c) un second promoteur lié à un gène codant pour un marqueur ou indicateur destiné à la sélection de l'avipoxvirus recombinant;
d) des séquences d'ADN de l'avipoxvirus adjacentes en 5' et 3' à la construction contenant les éléments a-c, les séquences de l'ADN de l'avipoxvirus adjacentes étant homologues à une région du génome viral où les éléments a-c doivent être insérés, les séquences adjacentes étant homologues au fragment BamHI J du génome de l'avipox ou à une partie de celui-ci suffisante pour permettre la recombinaison, la région étant telle que l'insertion d'un ADN étranger ne réduit pas notablement l'immunogénicité du virus recombinant dans l'animal hôte;
e) un réplicon destiné à la réplication du vecteur dans une cellule hôte procaryote; et
f) un gène de structure codant pour un marqueur ou indicateur destiné à la sélection du vecteur dans une cellule hôte procaryote, le gène étant sous le contrôle d'un promoteur procaryote.

14. Vecteur selon la revendication 13, dans lequel les promoteurs sont issus de l'avipoxvirus.

15. Vecteur selon la revendication 14, dans lequel le promoteur de l'avipoxvirus est choisi au sein du groupe constitué par les promoteurs C1 et C2, ou des versions modifiées de ceux-ci, possédant les séquences d'ADN présentées à la figure 5.

16. Vecteur selon la revendication 13, dans lequel la séquence d'ADN étranger est une séquence codant pour une protéine immunogène ou un fragment protéique d'un agent pathogène.

17. Vecteur selon la revendication 16, dans lequel l'agent pathogène est un parasite de l'espèce Eimeria.

18. Vecteur selon la revendication 17, dans lequel la protéine immunogène est la protéine GX3262 d' Eimeria tenella.

19. Vecteur selon la revendication 13, dans lequel le gène de structure ou le gène marqueur sous le contrôle du second promoteur est le gène lacZ.

20. Vecteur d'ADN selon la revendication 13, dans lequel les séquences adjacentes en 5' sont homologues aux séquences d'ADN du fragment BamHI J en 5' du site Bglll et les séquences adjacentes en 3' sont homologues aux séquences d'ADN du fragment BamHI J en 3' du site BgIII.

21. Avipoxvirus recombinant, intact sur le plan immunologique, produit en:
a) insérant une ou plusieurs séquences d'ADN étranger dans un vecteur selon la revendication 8; et en
b) exposant le vecteur contenant l'ADN étranger à une recombinaison in vivo avec l'avipoxvirus de manière à produire un avipoxvirus recombinant dans le génome duquel sont insérées des séquences d'ADN étranger et un promoteur de l'avipoxvirus capable de contrôler l'expression de ces séquences d'ADN étranger.

22. Avipoxvirus recombinant selon la revendication 21, dans lequel le promoteur de l'avipoxvirus est un promoteur choisi au sein du groupe constitué par les promoteurs C1, C2 et leurs versions modifiées possédant les séquences d'ADN présentées à la figure 5.

23. Avipoxvirus recombinant selon la revendication 21, dans lequel les séquences d'ADN étranger sont des séquences qui codent pour une ou plusieurs protéines immunogènes ou fragment protéiques d'un agent pathogène.

24. Avipoxvirus recombinant selon la revendication 23, dans lequel l'agent pathogène est un parasite de l'espèce Eimeria.

25. Avipoxvirus recombinant selon la revendication 24, dans lequel la protéine immunogène est la protéine GX3262 d' Eimeria tenella.

26. Utilisation d'un avipoxvirus recombinant capable d'exprimer un ADN étranger codant pour une ou plusieurs protéines immunogènes d'un agent pathogène, dans lequel l'ADN étranger est inséré dans une région du génome viral homologue au fragment BamHI J de l'avipoxvirus, dont l'insertion ne réduit pas considérablement l'immunogénicité du virus recombinant dans un animal hôte, l'expression de chaque protéine immunogène étant sous le contrôle d'un promoteur de l'avipoxvirus, pour la fabrication d'un vaccin destiné à vacciner des volailles ou un autre animal contre l'agent pathogène.

27. Utilisation selon la revendication 26, dans laquelle l'agent pathogène est un parasite de l'espèce Eimeria.

28. Utilisation selon la revendication 26, dans laquelle la protéine immunogène est la protéine GX3262 d' Eimeria tenella.
